# EUROPEAN PATENT APPLICATION

(11) **EP 0 700 912 A1**
(43) Date of publication of application: **13.03.1996**
(21) Application number: 94915286.2
(22) Date of filing: 24.05.1994
(51) Int. Cl.: C07D 413/04, C07D 417/04, A61K 31/435

(54) **QUINOLONE DERIVATIVE OR SALT THEREOF, AND ANTIBACTERIAL CONTAINING THE SAME**

(30) Priority: 24.05.1993 JP 121359/93
(71) Applicant: WAKUNAGA SEIYAKU KABUSHIKI KAISHA, Osaka-Shi Osaka 532 (JP); FUJISAWA PHARMACEUTICAL CO., LTD., Osaka-shi Osaka 541 (JP)
(72) Inventor: MARUYAMA, Shinobu, Kodacho, Takata-gun Hiroshima 729-69 (JP); KURAMOTO, Yasuhiro, Kodacho, Takata-gun, Hiroshi 729-64 (JP); HAYASHI, Norihiro, Kodacho, Takata-gun, Hiroshima 729-64 (JP); OHSHITA, Yoshihiro, Kodacho, Takata-gun, Hiroshima 729-64 (JP); HIRAO, Yuzo, Kodacho, Takata-gun, Hiroshima 729-64 (JP)
(74) Representative: Wächtershäuser, Günter, Prof. Dr.
(86) International application number: JP9400827
(87) International publication number: WO9427993

(57) **Abstract**

A quinolone derivative represented by general formula (1) or a salt thereof, and an antibacterial containing the same, wherein R¹ represents isoxazolyl or isothiazolyl each of which may be substituted; R² represents hydrogen, halogen, lower alkyl, hydroxy, amino or nitro; R³ represents hydrogen or halogen; and Y represents halogen, optionally substituted saturated cyclic amino or H₂N-(CH₂)ₘ-A-. This compound is useful as an antibacterial, can be used as medicines for humans and animals, drugs for fishes, pesticides and food preservative, and is expected to have an anti-HIV activity.

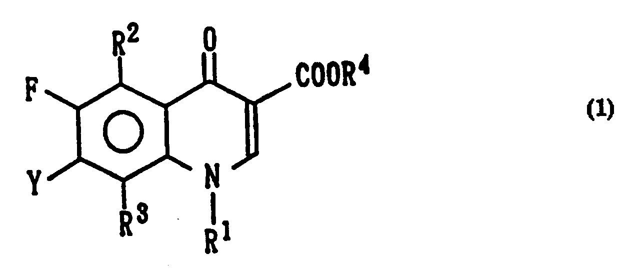

## Description

### Technical Field

The present invention relates to novel quinolone derivatives and salts thereof having excellent antibacterial activity and oral absorption, and antibacterial agents containing these compounds.

### Background Art

Among the compounds which have pyridonecarboxylic acid as a basic skeleton, many are known to be useful as synthetic antibacterial agents due to their excellent antibacterial activities and broad antibacterial spectrum. Mention may be given to norloxacin (Japanese Patent Laid-Open No. 141286/1978), enoxacin (Japanese Patent Laid-Open No. 31042/1980), ofloxacin (Japanese Patent Laid-Open No. 46986/1982), cyprofloxacin (Japanese Laid-Open No. 76667/1983) and the like, which are widely used in clinical application as therapeutic agents for infectious diseases.

These compounds are, however, still insufficient in terms of antibacterial activities, intestinal absorption, metabolic stability, side effects and the like. Hence, there has been a demand for the development of novel compounds which can satisfy these requirements.

### Disclosure of the Invention

In view of the foregoing circumstances, the present inventors have carried out an extensive investigation with a view toward obtaining clinically excellent synthetic antibacterial agents. As a result, it has been found that a compound represented by the formula (1) to be described below has excellent oral absorption, exhibits excellent antibacterial activities against gram negative and gram positive bacteria, and is therefore very useful as a synthetic antibacterial agent, leading to the completion of the invention.

The present invention therefore provides a quinolone derivative represented by the following formula (1): 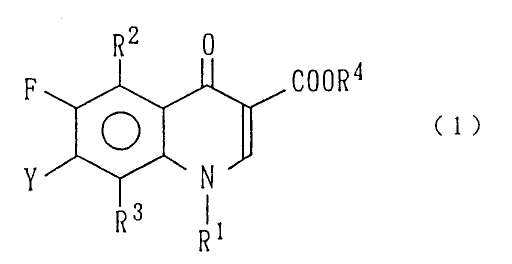
wherein R¹ represents a substituted or unsubstituted isoxazolyl group or a substituted or unsubstituted isothiazolyl group, R² represents a hydrogen atom, a halogen atom, a lower alkyl group, a hydroxyl group, an amino group or a nitro group, R³ represents a hydrogen atom or a halogen atom, R⁴ represents a hydrogen atom or a carboxyl-protecting group, and Y represents a halogen atom, a saturated cyclic amino group which may be substituted or a group H₂N-(CH₂)ₘ-A- in which A represents an oxygen or sulfur atom and m stands for 0-3; or a salt thereof.

The present invention also provides an antibacterial agent comprising as an effective ingredient a quinolone derivative represented by the above formula (1) or a salt thereof.

The present invention further provides a pharmaceutical composition comprising a quinolone derivative represented by the above formula (1) or a salt thereof, and a pharmaceutically-acceptable carrier.

### Best Modes for Carrying Out the Invention

The term "lower" as used for the description of each substituent which is contained in a quinolone derivative or a salt thereof (1) means a C₁₋₇, preferably C₁₋₅ group when the substituent represents a linear or a branched group, or a C₃₋₇ group when the substituent is a cyclic group.

Specific examples of the substituent of the isoxazolyl or isothiazolyl group represented by R¹ include lower alkyl groups such as methyl and ethyl, aryl groups such as phenyl, halogen atoms such as chlorine and fluorine, hydroxyl groups, lower alkoxyl groups, amino groups and nitro groups.

Specific examples of the substituted or unsubstituted isoxazolyl group represented by R¹ include 3-isoxazolyl, 4-isoxazolyl, 5-isoxazolyl, 4-methyl-3-isoxazolyl, 5-methyl-3-isoxazolyl, 3-methyl-5-isoxazolyl, 3,5-dimethyl-4-isoxazolyl, 4-chloro-3-isoxazolyl, 5-chloro-3-isoxazolyl and 3-chloro-5-isoxazolyl.

Specific examples of the substituted or unsubstituted isothiazolyl group represented by R¹ include 3-isothiazolyl, 4-isothiazolyl, 4-methyl-3-isothiazolyl, 3-methyl-5-isothiazolyl, 3-methyl-4-isothiazolyl, 4-chloro-3-isothiazolyl, 3-chloro-5-isothiazolyl and 3-chloro-4-isothiazolyl.

Illustrative examples of the lower alkyl group represented by R² include methyl, ethyl, n-propyl and t-butyl.

Examples of the halogen atom represented by R² or R³ include fluorine, chlorine and bromine, with fluorine being particularly preferred.

The carboxyl-protecting group represented by R⁴ is the ester residue of a carboxylic acid ester and means a desired group capable of undergoing relatively easy cleavage and yielding a corresponding free carboxyl group. Specific examples include those removable upon treatment under mild conditions such as hydrolysis or catalytic reduction, such as lower alkyl groups (e.g., methyl, ethyl, n-propyl, t-butyl, etc.), lower alkenyl groups (e.g., allyl, etc.), aralkyl groups (e.g., benzyl, etc.), and aryl groups (e.g., phenyl, etc.); and those readily eliminated *in vivo*, such as lower alkanoyloxy-lower alkyl groups (e.g., acetoxymethyl, pivaloyloxymethyl, etc.), lower alkoxycarbonyloxy-lower alkyl groups (e.g., methoxycarbonyloxymethyl, 1-ethoxycarbonyloxyethyl, etc.), lower alkoxymethyl groups (e.g., methoxymethyl, etc.), lactonyl groups (e.g., phthalidyl, etc.), di(lower alkyl)amino-lower alkyl groups (e.g., 1-dimethylaminoethyl, etc.), (5-methyl-2-oxo-1,3-dioxol-4-yl)methyl group, and the like.

Examples of the halogen atom represented by Y include those represented by R² or R³, with fluorine and chlorine being particularly preferred.

The saturated cyclic amino group which may be substituted may have, on its ring, further one or more hetero atoms such as nitrogen, oxygen, sulfur and/or the like, or carbonyl carbons. It may be mono, di- or tri-cyclic. When the groups are monocyclic, di-cyclic and tri-cyclic, 4-7 membered, 7-11 membered and 9-15 membered ones are preferred, respectively. Examples of such cyclic amino groups include saturated monocylic 3-7 membered amino groups having one nitrogen atom, such as azirydin-1-yl, azetidin-1-yl, pyrrolidin-1-yl, piperidino and perhydroazepin-1-yl; saturated monocyclic 3-7 membered amino groups having two nitrogen atoms, such as piperazin-1-yl and homopiperazin-1-yl; saturated monocyclic 3-7 membered amino groups having a nitrogen atom and also a hetero atom selected from oxygen or sulfur, such as oxazolidin-3-yl, isoxazolidin-2-yl, morpholino, 1,3-oxazin-3-yl, thiazolidin-1-yl, isothiazolidin-1-yl and thiomorpholin-1-yl; saturated di- or tri-cyclic amino groups such as tetrahydroquinolin-1-yl; and saturated 5-12 membered cyclic amino groups of a spiro or bridge type, such as 2,8-diazaspiro[4,4]nonan-2-yl, 7-azabicyclo[2,2,1]heptan-7-yl, 2,8-diazabicyclo[4,3,0]nonan-2-yl, 5-methyl-2,5-diazabicyclo[2,2,1]heptan-2-yl, 2,5-diazabicyclo[2,2,1]heptan-2-yl and 3,8-diazabicyclo[3,2,1]octan-3-yl.

Each atom of these cyclic amino groups may each be substituted by a suitable substituent. Examples of the substituent include lower alkyl groups, lower alkenyl groups, lower aralkyl groups, aryl groups, hydroxyl groups, hydroxy-lower alkyl groups, substituted or unsubstituted amino groups, substituted or unsubstituted amino-lower alkyl groups, cyclic amino groups exemplified above, alkoxyl groups, alkoxy-lower alkyl groups, aryloxy groups, halogen atoms, halo-lower alkyl groups, acyloxy groups, acyloxy-lower alkyl groups, acyl groups, carboxyl groups, carboxy-lower alkyl groups, alkoxycarbonyl-lower alkyl groups, mercapto groups, lower alkylthio groups, cyano groups, cyano-lower alkyl groups, nitro groups and heterocyclic groups such as pyridinyl groups.

Examples of the lower alkyl groups include methyl, ethyl and n-propyl; those of the lower alkenyl groups include vinyl and allyl; those of the lower aralkyl groups include benzyl and 1-phenylethyl; those of the aryl groups include phenyl; those of the hydroxy-lower alkyl groups include hydroxymethyl, hyroxyethyl and hydroxypropyl; those of the amino-lower alkyl groups include aminomethyl, 1-aminoethyl, 2-aminoethyl and 1-amino-1-methylethyl; those of the alkoxyl groups include methoxy, ethoxy and n-propoxy; those of the alkoxy-lower alkyl groups include methoxymethyl and ethoxymethyl; those of the halogen atoms include fluorine, chlorine and bromine; those of the halo-lower alkyl groups include fluoromethyl and trifluoromethyl; those of the acyloxy groups include lower-alkanoyloxy-lower alkyl and aromatic acyloxy-lower alkyl, e.g., acetoxy and benzoyloxy; those of the acyloxy-lower alkyl groups include acetoxymethyl and benzoyloxy; those of the acyl groups include lower alkanoyl such as formyl and acetyl, lower alkoxycarbonyl such as methoxycarbonyl and ethoxycarbonyl, and aromatic acyl such as benzoyl and phenoxycarbonyl; those of the carboxy-lower alkyl groups include carboxymethyl and carboxyethyl; those of the alkoxycarbonyl-lower alkyl groups include methoxycarbonylmethyl and t-butoxycarbonylmethyl; and those of the lower alkylthio groups include methylthio and ethylthio.

Illustrative of the substituent of the substituted amino group or the substituted amino-lower alkyl group include lower alkyl groups (e.g., methyl, ethyl, etc.), cyclo-lower alkyl groups (e.g., cyclopropyl, cyclobutyl, cyclopentyl, etc.), lower alkenyl groups (e.g., vinyl, allyl, etc.), lower aralkyl groups (e.g., benzyl, 1-phenylethyl, etc.), aryl groups (e.g., phenyl, etc.), acyl groups (e.g., lower alkanoyl groups such as formyl and acetyl, lower alkoxycarbonyl groups such as methoxycarbonyl and ethoxycarbonyl, etc.), amino acid residues or peptide residues (e.g., glycyl, leucyl, valyl, alanyl, phenylalanyl, alanyl-alanyl, glycyl-valyl and glycyl-glycyl-valyl, etc.), amino acid residues or peptide residues having the above-described functional group protected with a protecting group such as acyl, lower aralkyl or the like commonly used in the peptide chemistry; and cyclic amino groups. The substituent can be selected freely from 1-2 substituents of the same type or different types. Compounds protected with such an amino acid residue or peptide residue is expected to have an improved water solubility.

Particularly preferred examples of the substituted amino group or substituted amino-lower alkyl group include methylamino, ethylamino, dimethylamino, methylaminomethyl, ethylaminomethyl, dimethylaminomethyl, glycyl-amino, leucyl-amino, valyl-amino, alanyl-amino and alanyl-alanyl-amino.

Among the saturated cyclic amino groups, preferred examples include those represented by the following formulas (a) and (b): 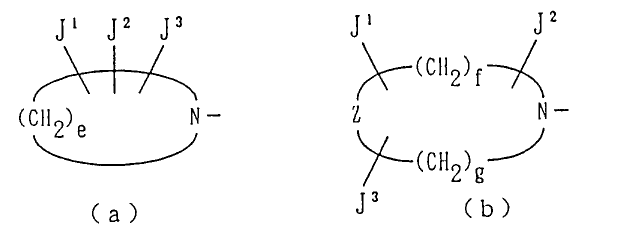
wherein Z represents an oxygen atom, a sulfur atom, -NR⁵ or -CONR⁵ in which R⁵ represents a hydrogen atom, a hydroxyl group, a lower alkyl group, a cyclo-lower alkyl group, an aralkyl group, an alkenyl group, an acyl group or a hydroxyl-lower alkyl group, e stands for 3-5, f stands for 1-3, g stands for 0-2, J¹, J² and J³ are the same or different and individually represent a hydrogen atom, a lower alkyl group, a lower alkenyl group, a lower aralkyl group, an aryl group, a hydroxyl group, a hydroxy-lower alkyl group, a substituted or unsubstituted amino group, a substituted or unsubstituted amino-lower alkyl group, a pyrrolidinyl group, a piperidino group, an azetidinyl group, an alkoxyl group, an alkoxyl-lower alkyl group, a halogen atom, a halo-lower alkyl group, an acyloxy group, an acyloxy-lower alkyl group, an acyl group, a carboxyl group, a carboxy-lower alkyl group, an alkoxycarbonyl-lower alkyl group, a mercapto group, a lower alkylthio group, a cyano group or a nitro group.

The definition of the substituents of J¹, J² and J³ and preferred examples of them are the same as those described in relation to the substituents for the above cyclic amino group.

Examples of the heterocyclic ring group represented by the formula (a) include azetidinyl, pyrrolidinyl and piperidino; and those of the heterocyclic ring group represented by the formula (b) include piperazinyl, morpholino, thiomorpholino, homopiperazinyl, thiazolidinyl, oxazolidinyl and 3-oxo-1-piperazinyl.

Particularly preferred examples of the groups represented by the formulas (a) and (b) include 3-hydroxyazetidinyl, 3-aminoazetidinyl, 3-(N-t-butoxycarbonylamino)azetidinyl, 3-acetylaminoazetidinyl, 3-methylaminoazetidinyl, 3-dimethylaminoazetidinyl, 3-methylazetidinyl, 3-amino-2-methylazetidinyl, 3-phenyl-3-hydroxyazetidinyl, 3-phenyl-3-aminoazetidinyl, pyrrolidinyl, 3-hydroxypyrrolidinyl, 3,4-dihydroxypyrrolidinyl, 3-methoxypyrrolidinyl, 3-methylpyrrolidinyl, 3-hydroxy-4-methyl-pyrrolidinyl, 3-aminopyrrolidinyl, 3-methylaminopyrrolidinyl, 3-dimethylaminopyrrolidinyl, 3-ethylaminopyrrolidinyl, 3-diethylaminopyrrolidinyl, 3-acetylaminopyrrolidinyl, 3-t-butoxycarbonylaminopyrrolidinyl, 3-(N-acetyl)methylaminopyrrolidinyl, 3-(t-butoxycarbonyl)methylaminopyrrolidinyl, 3-aminomethylpyrrolidinyl, 3-methylaminomethylpyrrolidinyl, 3-dimethylaminomethylpyrrolidinyl, 3-ethylaminomethylpyrrolidinyl, 3-diethylaminomethylpyrrolidinyl, 3-(N-acetyl)aminomethylpyrrolidinyl, 3-(t-butoxycarbonyl)aminomethylpyrrolidinyl, 3-(N-acetyl)methylaminomethylpyrrolidinyl, 3-(t-butoxycarbonyl)methylaminomethylpyrrolidinyl, 3-(1-aminoethyl)pyrrolidinyl, 3-(2-aminoethyl)pyrrolidinyl, 3-(1-amino-1-methylethyl)pyrrolidinyl, 3-(1-methylaminoethyl)pyrrolidinyl, 3-(1-dimethylaminoethyl)pyrrolidinyl, 3-amino-3-phenylpyrrolidinyl, 3-hydroxy-3-phenylpyrrolidinyl, 3-amino-3-methylpyrrolidinyl, 3-amino-4-methylpyrrolidinyl, 3-amino-5-methylpyrrolidinyl, 3-methylamino-4-methylpyrrolidinyl, 3-dimethylamino-4-methylpyrrolidinyl, 3-ethylamino-4-methylpyrrolidinyl, 3-diethylamino-3-methylpyrrolidinyl, 3-diethylamino-4-methylpyrrodinyl, 3-aminomethyl-4-methylpyrrolidinyl, 3-methylaminomethyl-4-methylpyrrolidinyl, 3-dimethylaminomethyl-4-methylpyrrolidinyl, 3-ethylaminomethyl-4-methylpyrrolidinyl, 3-(1-aminoethyl)-4-methylpyrrolidinyl, 3-(2-aminoethyl)-4-methylpyrrolidinyl, 3-amino-4-ethylpyrrolidinyl, 3-methylamino-4-ethylpyrrolidinyl, 3-dimethylamino-4-ethylpyrrolidinyl, 3-ethylamino-4-ethylpyrrolidinyl, 3-diethylamino-4-ethylpyrrolidinyl, 3-aminomethyl-4-ethylpyrrolidinyl, 3-methylaminomethyl-4-ethylpyrrolidinyl, 3-dimethylaminomethyl-4-ethylpyrrolidinyl, 3-amino-3-methylpyrrolidinyl, 3-methylamino-3-methylpyrrolidinyl, 3-dimethylamino-3-methylpyrrolidinyl, 3-amino-3,4,-dimethylpyrrolidinyl, 3-amino-4,4-dimethylpyrrolidinyl, 3-amino-4,5-dimethylpyrrolidinyl, 3-amino-2,4-dimethylpyrrolidinyl, 3-methylamino-3,4-dimethylpyrrolidinyl, 2-methyl-3-aminopyrrolidinyl, 2-methyl-3-dimethylaminopyrrolidinyl, 3-amino-4-vinylpyrrolidinyl, 3-amino-4-methoxypyrrolidinyl, 3-amino-4-methoxymethylpyrrolidinyl, 3-methylamino-4-methoxypyrrolidinyl, 3-dimethylamino-4-methoxypyrrolidinyl, 3-ethylamino-4-methoxypyrrolidinyl, 3-diethylamino-4-methoxypyrrolidinyl, 3-benzylamino-4-methoxypyrrolidinyl, 3-aminomethyl-4-methoxypyrrolidinyl, 3-methylaminomethyl-4-methoxypyrrolidinyl, 3-dimethylaminomethyl-4-methoxypyrrolidinyl, 3-ethylaminomethyl-4-methoxypyrrolidinyl, 3-aminomethyl-3-methoxypyrrolidinyl, 3-methylaminomethyl-3-methoxypyrrolidinyl, 3-dimethylaminomethyl-3-methoxypyrrolidinyl, 3-amino-4-ethoxypyrrolidinyl, 3-methylamino-4-ethoxypyrrolidinyl, 3-dimethylamino-4-ethoxypyrrolidinyl, 3-methylamino-4-ethoxypyrrolidinyl, 3-aminomethyl-4-ethoxypyrrolidinyl, 3-dimethylaminomethyl-4-ethoxypyrrolidinyl, 3-amino-4-aminocarbamoylpyrrolidinyl, 3-amino-4-dimethylaminocarbamoylpyrrolidinyl, 3-amino-4-hydroxypyrrolidinyl, 3-amino-4-hydroxymethylpyrrolidinyl, 3-amino-4-hydroxyethylpyrrolidinyl, 3-amino-4-methyl-4-hydroxymethylpyrrolidinyl, 3-aminomethyl-4-hydroxypyrrolidinyl, 3-dimethylaminomethyl-4-hydroxypyrrolidinyl, 3,4-dihydroxypyrrolidinyl, 3,4-dimethoxypyrrolidinyl, 3-hydroxy-4-methylpyrrolidinyl, 3-amino-4-fluoropyrrolidinyl, 3-amino-4-fluoromethylpyrrodinyl, 3-amino-4-trifluoromethylpyrrolidinyl, 3-methylamino-4-fluoropyrrolidinyl, 3-dimethylamino-4-fluoropyrrolidinyl, 3-aminomethyl-4-fluoropyrrolidinyl, 3-methylaminomethyl-4-fluoropyrrolidinyl, 3-dimethylaminomethyl-4-fluoropyrrolidinyl, 3-methylamino-4-chloropyrrolidinyl, 3-aminomethyl-4-chloropyrrolidinyl, 3-methylaminomethyl-4-chloropyrrolidinyl, 3-(2-hydroxyethyl)aminomethylpyrrolidinyl, 3-(2-fluoroethyl)aminomethylpyrrolidinyl, 3-amino-4-methylthiopyrrolidinyl, 3-amino-4-methylsulfinylpyrrolidinyl, 3-formimidoylaminopyrrolidinyl, 3-(2-dimethylhydrazino)pyrrolidinyl, 3-amino-4-methylenepyrrolidinyl, 3-(t-butoxycarbonylaminoacetyl)amino-4-methylpyrrolidinyl, 3-aminoacetylamino-4-methylpyrrolidinyl, 3-(2-aminopropanoyl)amino-4-methylpyrrolidinyl, 3-(2-amino-3-phenylpropanoyl)amino-4-methylpyrrolidinyl, 3-(2-benzyloxycarbonylamino-3-methylbutanoylamino-4-methylpyrrolidinyl, 3-(2-amino-3-methylbutanoyl)amino-4-methylpyrrolidinyl, 3-(2-amino-2-methylpropanoyl)amino-4-methylpyrrolidinyl, 7-amino-5-azaspiro[2.4]heptan-5-yl, piperazinyl, 4-methylpiperazinyl, 3-methylpiperazinyl, 2-methylpiperazinyl, 3,4-dimethylpiperazinyl, 3,5-dimethylpiperazinyl, 3,3-dimethylpiperazinyl, 3,4,5-trimethylpiperazinyl, 4-ethoxycarbonylpiperazinyl, 4-t-butoxycarbonylpiperazinyl, 4-acetylpiperazinyl, 4-benzyloxycarbonylpiperazinyl, 4-ethylpiperazinyl, 3,4-diethylpiperazinyl, 3,4,5-triethylpiperazinyl, 4-ethyl-3,5-dimethylpiperazinyl, 3-methyl-4-acetylpiperazinyl, 3-methyl-4-t-butoxycarbonylpiperazinyl, 4-benzylpiperazinyl, 4-n-propylpiperazinyl, 4-isopropylpiperazinyl, 4-t-butylpiperazinyl, 4-cyclopropylpiperazinyl, 4-cyclopentylpiperazinyl, 4-cyclopropylmethylpiperazinyl, 4-phenylpiperazinyl-4-(p-dimethylaminophenyl)piperazinyl, 4-(p-methoxyphenyl)piperazinyl, 4-(p-fluorophenyl)piperazinyl, 3-phenylpiperazinyl, 3-(p-fluorophenyl)piperazinyl, 3-(p-chlorophenyl)piperazinyl, 3-(p-hydroxyphenyl)piperazinyl, 3-(p-methylphenyl)piperazinyl, 4-hydroxyethylpiperazinyl, 4-aminoethylpiperazinyl, 4-allylpiperazinyl, 4-cinnamylpiperazinyl, 4-cyanoethylpiperazinyl, 4-carboxyethylpiperazinyl, 4-carboxymethylpiperazinyl, 4-(1,2-dicarboxyethyl)piperazinyl, 4-hydroxypiperazinyl, 3-fluoromethylpiperazinyl, 3-trifluoromethylpiperazinyl, 4-formimidoylpiperazinyl, 4-acetoimidoylpiperazinyl, piperidino, 4-aminopiperidino, 4-dimethylaminopiperidino, 4-hydroxypiperidino, morpholino, 2-aminomethylmorpholino, 2-methylaminomorpholino, 2-dimethylaminomorpholino, thiomorpholino, homopiperazinyl, 4-methylhomopiperazinyl, thiazolydinyl and oxazolidinyl.

The quinolone derivatives or salts thereof (1) can be converted into both acid addition salts and base addition salts. Incidentally, these salts include those formed into chelete salts with a boron compound. Exemplary acid addition salts include (a) salts with mineral acids such as hydrochloric acid and sulfuric acid, (b) salts with organic carboxylic acids such as formic acid, citric acid, trichloroacetic acid, trifluoroacetic acid, fumaric acid and maleic acid, and (c) salts with sulfonic acids such as methanesulfonic acid, benzenesulfonic acid, p-toluenesulfonic acid, mesitylenesulfonic acid and naphthalenesulfonic acid. On the other hand, illustrative base addition salts include (a') salts with alkali metals such as sodium and potassium, (b') salts with alkaline earth metals such as calcium and magnesium, (c') ammonium salts, (d') salts with nitrogen-containing organic bases such as trimethylamine, triethylamine, tributylamine, pyridine, N,N-dimethylaniline, N-methylpiperidine, N-methylmorpholine, diethylamine, cyclohexylamine, procaine, dibenzylamine, N-benzyl-β-phenethylamine, 1-ephenamine and N,N'-dibenzylethylenediamine. Examples of the boron compounds include boron halides such as boron fluoride, and lower acyloxy borons such as acetoxy boron.

The quinolone derivatives or salts thereof (1) can exist not only in unsolvated forms but also in hydrated or solvated forms. The compounds according to the present invention therefore embrace those in any crystalline forms and their hydrated and solvated forms.

The quinolone derivatives or salts thereof (1) include those containing an asymmetric carbon atom so that they can exist as optically active substances. These optically active substances are also embraced in the compounds of the present invention. The compounds (1) also include those containing two or more asymmetric carbon atoms. They can exist as different stereoisomers (cis-form, trans-form). These stereoisomers are also included in the compounds of the present invention.

The quinolone derivatives or salts thereof (1) can each be prepared by any one of the processes suited for the types of its substituents. Preferred are the preparation processes shown below.

### (Process-1)

Among the compounds represented by formula (1), those in which Y is halogen atom can be prepared, for example, by the series of steps shown in the following process 1. 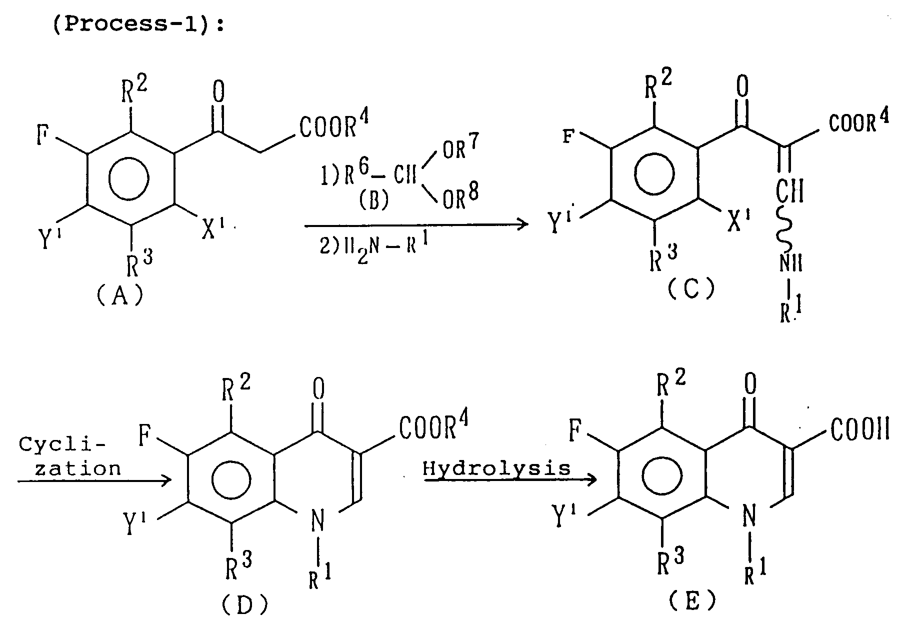
wherein X¹ and Y¹ each represents a halogen atom, R⁶ represents a lower alkoxyl group or 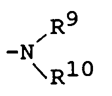
in which R⁹ and R¹⁰ each represents a lower alkyl group, R⁷ and R⁸ each represents a lower alkyl group and R¹ to R⁴ have the same meanings as defined above.

Namely, Compound (C) can be obtained by reacting, in acetic anhydride, Compound (A) with an orthoformic acid ester (B) such as ethyl orthoformate or methyl orthoformate, and then reacting the reaction product with Compound H₂N-R¹. The reaction of Compound (A) and the orthoformic acid ester may ordinarily be conducted at 0-160°C, preferably 50-150°C. The reaction time may generally be 10 minutes to 48 hours, preferably 1 hour to 10 hours. The orthoformic acid ester may be used in at least an equimolar amount, preferably in a molar amount about 1-10 times, both relative to Compound (A).

The reaction with the above compound H₂N-R¹ may be conducted in a suitable organic solvent. Any solvent can be used here as long as it does not affect the reaction. Examples of such a solvent include aromatic hydrocarbons such as benzene, toluene and xylene; ethers such as diethyl ether, tetrahydrofuran, dioxane, monoglyme and diglyme; aliphatic hydrocarbons such as pentane, hexane, heptane and ligroin; halogenated hydrocarbons such as methylene chloride, chloroform and carbon tetrachloride; aprotonic polar solvents such as dimethylformamide and dimethyl sulfoxide, and alcohols such as methanol, ethanol and propanol. This reaction may ordinarily be conducted at 0-150°C, preferably 0-100°C. The reaction time may generally be 10 minutes to 48 hours. The compound H₂N-R¹ may be used in at least an equimolar amount, preferably in a molar amount about 1-2 times, both relative to Compound (A).

As an alternative, Compound (C) can be obtained by reacting Compound (A) with an acetal such as N,N-dimethylformamide dimethyl acetal or N,N-dimethylformamide diethyl acetal and then reacting the reaction product with the compound H₂N-R¹. Any solvent can be used for the reaction with the acetal as long as it is inert to the reaction. For example, those exemplified above can be used as a solvent. This reaction may ordinarily be conducted at 0-150°C, preferably room temperature to 100°C. The reaction time may generally be 10 minutes to 48 hours, preferably 1-10 hours.

Compound (D) can be obtained by subjecting Compound (C) to cyclization reaction. This reaction may be conducted in a suitable solvent in the presence of a basic compound. Any solvent can be used in this reaction so far as it does not affect the reaction. Examples of such a solvent include aromatic hydrocarbons such as benzene, toluene and xylene; ethers such as diethyl ether, tetrahydrofuran, dioxane and monoglyme; halogenated hydrocarbons such as methylene chloride, chloroform and carbon tetrachloride; alcohols such as methanol, ethanol, propanol and butanol; and aprotonic polar solvents such as dimethylformamide and dimethylsulfoxide. Illustrative basic compounds include alkali metals such as metallic sodium and metallic potassium; metal hydrides such as sodium hydride and calcium hydride; inorganic bases such as sodium hydroxide, potassium hydroxide and sodium carbonate; alkoxides such as sodium methoxide, sodium ethoxide and potassium-t-butoxide; metal fluorides such as sodium fluoride and potassium fluoride; and organic bases such as triethylamine, 1,8-diazabicyclo[5.4.0]undecene (DBU). The reaction temperature may ordinarily range from 0°C to 200°C, preferably from room temperature to 180°C. The reaction may generally be completed in 5 minutes to 24 hours. The basic compound may be used in at least an equimolar amount, preferably in a molar amount about 1-2 times, both relative to Compound (C).

If desired, Compound (E) can be obtained by the hydrolysis of Compound (D). This hydrolysis reaction can be conducted under any reaction conditions usable in the ordinary hydrolysis reaction. This reaction may be conducted, for example, in the presence of a basic compound such as sodium hydroxide, potassium hydroxide, sodium carbonate or potassium carbonate; a mineral acid such as hydrochloric acid, sulfuric acid or hydrobromic acid; or an organic acid such as p-toluenesulfonic acid and in a solvent such as water; an alcohol, e.g., methanol, ethanol or propanol; an ether, e.g., tetrahydrofuran or dioxane; a ketone, e.g., acetone or methyl ethyl ketone; acetic acid or a mixed solvent thereof. This reaction may be conducted ordinarily at room temperature to 180°C, preferably room temperature to 140°C. The reaction time may generally range from 1 to 24 hours.

### (Process-2)

Among the compounds represented by the formula (1), those having as Y a substituted or unsubstituted cyclic amino group or a group represented by the formula H₂N-(CH₂)ₘ-A- in which A and m have the same meanings as defined above, can be prepared, for example, in accordance with Process-2 shown by the following reaction scheme. 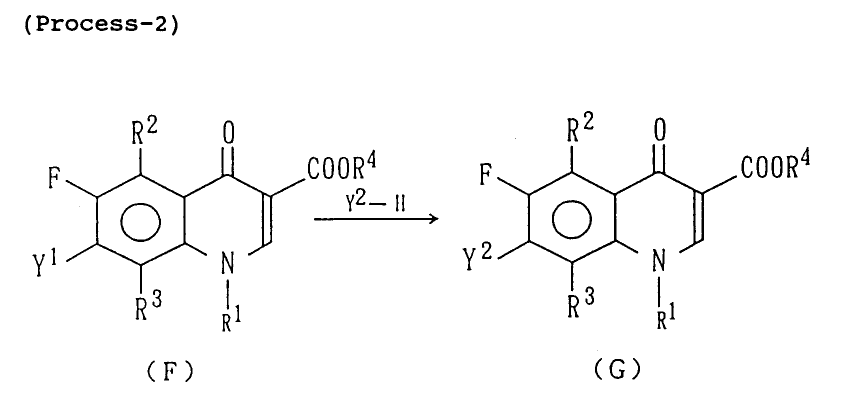
wherein Y² represents a substituted or unsubstituted cyclic amino group or a group H₂N-(CH₂)ₘ-A- in which A and m have the same meanings as defined above, and R¹ to R⁴ and Y¹ each has the same meaning as defined above.

Namely, Compound (G) can be obtained by reacting Compound (F) which has been obtained in Process-1 with a compound represented by the formula Y²-H.

This reaction may be conducted at room temperature to 160°C in a solvent which does not affect the reaction such as an aromatic hydrocarbon, e.g., benzene, toluene or xylene; an alcohol, e.g., methanol or ethanol, an ether, e.g., tetrahyrodofuran; dioxane or monoglyme, a halogenated hydrocarbon, e.g., methylene chloride, chloroform or carbon tetrachloride, aprotonic polar solvent, e.g., dimethylformamide, dimethyl sulfoxide or N-methylpyrrolidone; acetonitrile or pyridine, optionally in the presence of a neutralizing agent such as sodium carbonate, calcium carbonate, sodium bicarbonate, triethylamine or 1,8-diazabicyclo[5.4.0]undecene (DBU). The reaction time may range from several minutes to 48 hours, preferably 10 minutes to 24 hours. It is desired to use Y²-H in at least an equimolar amount, preferably in a molar amount of about 1-5 times, each relative to Compound (F).

When starting compounds used in the above Process 1 or 2 contain an amino group, imino group, hydroxyl group, mercapto group, carboxyl group or the like which does not take part in the reaction, they may be used in a form with the above group being protected. After the completion of the reaction, the protecting group may be eliminated in a manner known *per se* in the art. Any group can be used as a protecting group insofar as it can be eliminated without destroying the structure of the invention compound to be formed by the reaction. Groups usually employed in the field of peptide, aminosaccharide or nucleic acid chemistry can be used.

The starting compound (A) can be prepared by one of the processes described in the following documents or by a similar process.
1) J. Heterocyclic Chem. **22**, 1033(1985)
2) Liebigs Ann. Chem. 29(1987)
3) J. Med. Chem. **31**, 911(1988)
4) J. Org. Chem. **35**, 930(1970)
5) Japanese Patent Laid-Open No. 246541/1987
6) Japanese Patent Laid-Open No. 26272/1987
7) Japanese Patent Laid-Open No. 145268/1988
8) J. Med. Chem. **29**, 2363(1986)
9) J. Fluorin Chem. **28**, 361(1985)
10) Japanese Patent Laid-Open No. 198664/1988
11) Japanese Patent Laid-Open No. 264461/1988
12) Japanese Patent Laid-Open No. 104974/1988
13) European Patent Application No. 230948
14) Japanese Patent Laid-Open No. 282384/1990
15) Japanese Publication No. 502452/1991
16) J. Het. Chem. **27**, 1609(1990)

The compounds of the present invention thus obtained are isolated and purified in a manner known *per se* in the art. They are obtained in the form of a salt, free carboxylic acid or free amine, depending on the conditions for isolation and purification. They can, however, be converted mutually from one of these forms into another one, whereby the compound of the present invention can be prepared in a desired form.

When the compound (1) of the present invention is used as an antibacterial agent, it can be formulated, together with a pharmaceutically-acceptable carrier, into a pharmaceutical composition for parenteral administration such as injection, rectal administration or ophthalmic administration or for oral administration in the form of a solid or a liquid.

Pharmaceutical compositions of this invention for use as injections can take the form of pharmaceutically-acceptable germ-free water, non-aqueous solutions, suspensions or emulsions. Exemplary suitable non-aqueous carriers, diluents, solvents and vehicles include propylene glycol, polyethylene glycol, vegetable oils such as olive oil, and injectable organic esters such as ethyl oleate. These preparations can contain one or more auxiliary agents, for example, antiseptics, wetting agents, emulsifiers and dispersants. These compositions can be sterilized, for example, by filtering them through a bacterial filter or by mixing, immediately before use, a sterilizing agent or a sterilizing agent in the form of a germ-free solid composition soluble in sterilized water or one of some other media which can be sterilized and injected.

Preparations for ophthalmic administration can each preferably contain, in addition to compounds of the present invention, a solubilizing agent, preservative, isotonic agent, thickener and the like.

Exemplary solid preparations for oral administration include capsules, tablets, pills, powders, granules, etc. Upon formulation of these solid preparations, the compounds according to the present invention are generally mixed with at least one inert extender such as sucrose, lactose or starch. One or more materials other than inert extenders, for example, a lubricant such as magnesium stearate can also be incorporated in the preparations upon formulation of the latter in a usual manner. A buffer can also be incorporated in the case of capsules, tablets and pills. Tablets and pills can be applied with an enteric coating.

Illustrative liquid preparations for oral administration include pharmaceutically-acceptable emulsions, solutions, suspensions, syrups and elixirs, which contain an inert diluent employed commonly by those skilled in the art, for example, water. In addition to such an inert diluent, the liquid preparations can also be added with one or more auxiliary agents, for example, wetting agents, emulsifiers, suspending agents, sweetening agents, seasoning agents and perfumes.

Preparations for rectal administration are preferred to contain an excipient such as cacao butter or suppository wax in addition to a compound according to the present invention.

The dosage of the compound (1) according to the present invention depends on the properties of the compound to be administered, the administration route, the desired treatment term and other factors. It generally ranges from about 0.1 mg/kg to 1000 mg/kg per day, with about 1-100 mg/kg per day being preferred especially. If desired, this daily dosage can be administered in 2-4 portions.

The present invention will hereinafter be described more specifically by Examples. It should however be borne in mind that the present invention is not limited to or by the following Examples.

### Example 1

### Synthesis of ethyl 2-(2,4,5-trifluorobenzoyl)-3-(isoxazol-3-ylamino)acrylate (Compound No. 1)

Ethyl 2,4,5-trifluorobenzoylacetate (2.00 g), 1.81 g of ethyl orthoformate and 3.73 g of acetic anhydride were combined, followed by heating at 130°C for 3 hours. Excess ethyl orthoformate and acetic anhydride were distilled off and the residue was dissolved in 30 mℓ of chloroform. To the resulting solution, a solution of 683 mg of 3-aminoisoxazole in 30 mℓ of chloroform was added, followed by stirring at room temperature for 4 days. The solvent was then distilled off. The residue was purified using a silica gel column (hexane:chloroform = 1:2), whereby 2.69 g of the title compound were obtained as pale yellow needle crystals.
- Melting point:: 72.5-76.5°C
- ¹H-NMR(CDCℓ₃)δ;: 1.02(t,J=7Hz) and 1.16(t,J=7Hz) for 3H, 4.08-4.29(m,2H), 6.36(d,J=1.5Hz) and 6.37(d,J=1.5Hz) for 1H, 6.87-7.01(m,1H), 7.31-7.39(m) and 7.44-7.59(m) for 1H, 8.30(d,J=13.2Hz) and 8.58(d,J=12.7Hz) for 1H, 8.36(d,J=1.5Hz) and 8.39(d,J=1.5Hz) for 1H.

### Example 2

### Synthesis of ethyl 6,7-difluoro-1-(isoxazol-3-yl)-1,4-dihydro-4-oxoquinoline-3-carboxylate (Compound No. 2)

In 30 mℓ of dimethylformamide, 2.07 g of Compound No. 1 were dissolved. To the resulting solution, 925 mg of potassium carbonate were added, followed by stirring at 90°C for 10 minutes. After an aqueous solution of citric acid was added to the reaction mixture to neutralize the same, the solvent was distilled off. The residue was suspended in water, followed by collection through filtration. The solid so obtained was washed with water, ethanol and diethyl ether, whereby 1.29 g of the title compound was obtained as a colorless solid.
- Melting point:: 230-237°C
- ¹H-NMR(CDCℓ₃)δ;: 1.40(t,J=7Hz,3H), 4.40(q,J=7Hz,2H), 6.76(d,J=1.5Hz,1H), 7.33(dd,J=6.4Hz,10.8Hz,1H), 8.29(dd,J=8.8Hz,10.3Hz,1H), 8.56(s,1H), 8.76(d,J=1.5Hz,1H)

### Example 3

### Synthesis of 6,7-difluoro-1-(isoxazol-3-yl)-1,4-dihydro-4-oxoquinoline-3-carboxylic acid (Compound No. 3)

To 1.35 g of Compound No. 2, 40 ℓ of acetic acid and 20 mℓ of 6N hydrochloric acid were added, followed by stirring at 110°C for one hour. The reaction mixture was allowed to cool down, whereby crystals precipitated. Water was added to the reaction mixture to cause further precipitation. The crystals so precipitated were collected by filtration, followed by washing with water, ethanol and diethyl ether, whereby 1.17 g of the title compound were obtained as pale orange needle crystals.
- Melting point:: 236-237°C
- ¹H-NMR(DMSO-d₆)δ;: 7.29(d,J=2.0Hz,1H), 7.77(dd,J=6.4Hz,11.2Hz,1H), 8.35(dd,J=8.8Hz,10.7Hz,1H), 8.95(s,1H), 9.36(d,J=2.0Hz,1H)

### Example 4

### Synthesis of 7-{3(S)-aminopyrrolidin-1-yl}-6-fluoro-1-(isoxazol-3-yl)-1,4-dihydro-4-oxoquinoline-3- carboxylic acid hydrochloride (Compound No. 4)

In 5 mℓ of acetonitrile, 50 mg of Compound No. 3 were suspended. To the resulting suspension, 23 mg of 3-(S)-aminopyrrolidine and 41 mg of triethylamine were added, followed by stirring at 80 °C for one hour. The precipitate so obtained was collected by filtration, washed with ethanol and then dissolved in 6N hydrochloric acid to obtain its hydrochloride. The solvent was distilled off. The residue was then suspended in diethyl ether, followed by collection through filtration, whereby 64 mg of the title compound was obtained as a pale yellow solid.
- Melting point:: 213-225°C
- ¹H-NMR(DMSO-d₆)δ;: 1.94-2.18(brs,1H), 2.18-2.37(brs,1H), 3.76-3.99(m,2H), 6.43(d,J=6.8Hz,1H), 7.33(d,J=2.0Hz,1H), 7.92(d,J=14.2Hz,1H), 8.1-8.7(br,3H), 8.76(s,1H), 9.38(d,J=2.0Hz,1H)

### Example 5

In a similar manner to Example 4, Compounds Nos. 5-31 shown in Tables 1-1 to 1-6 were synthesized.

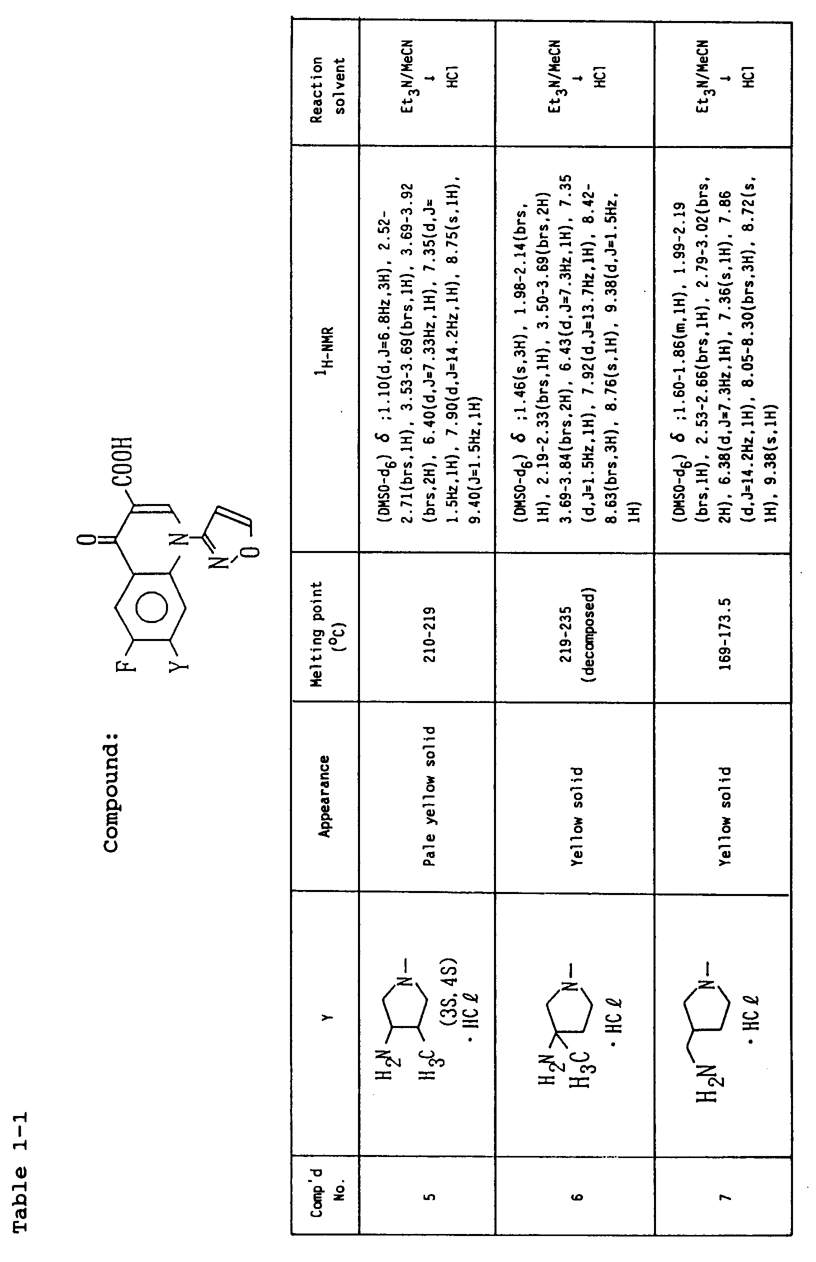

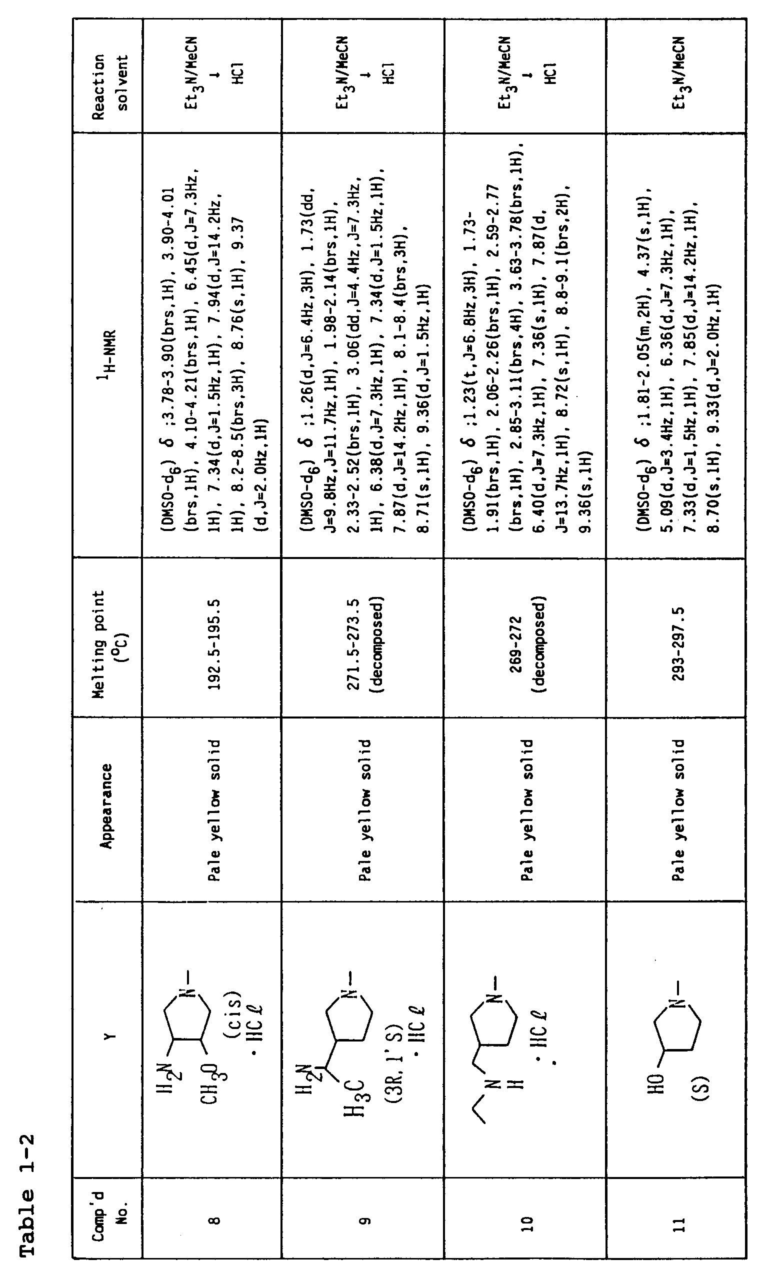

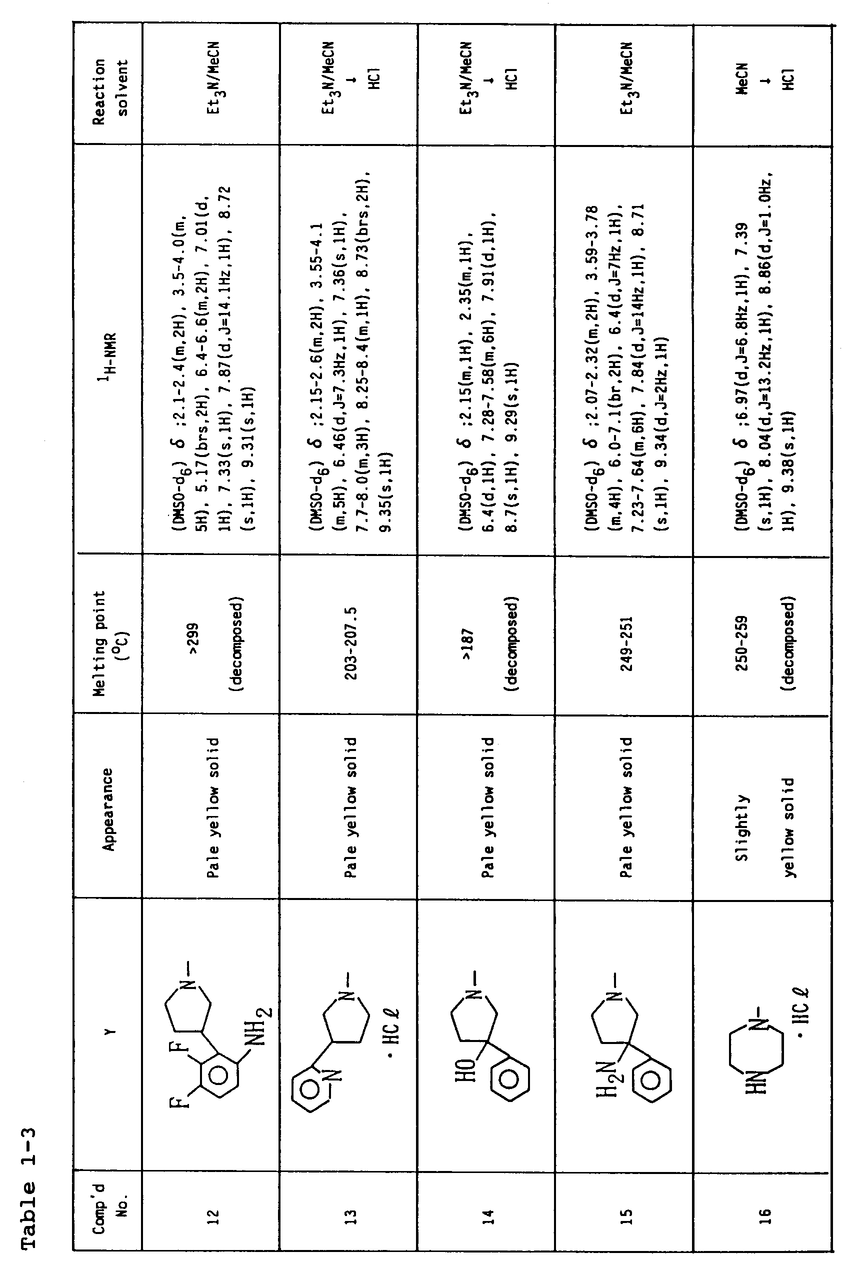

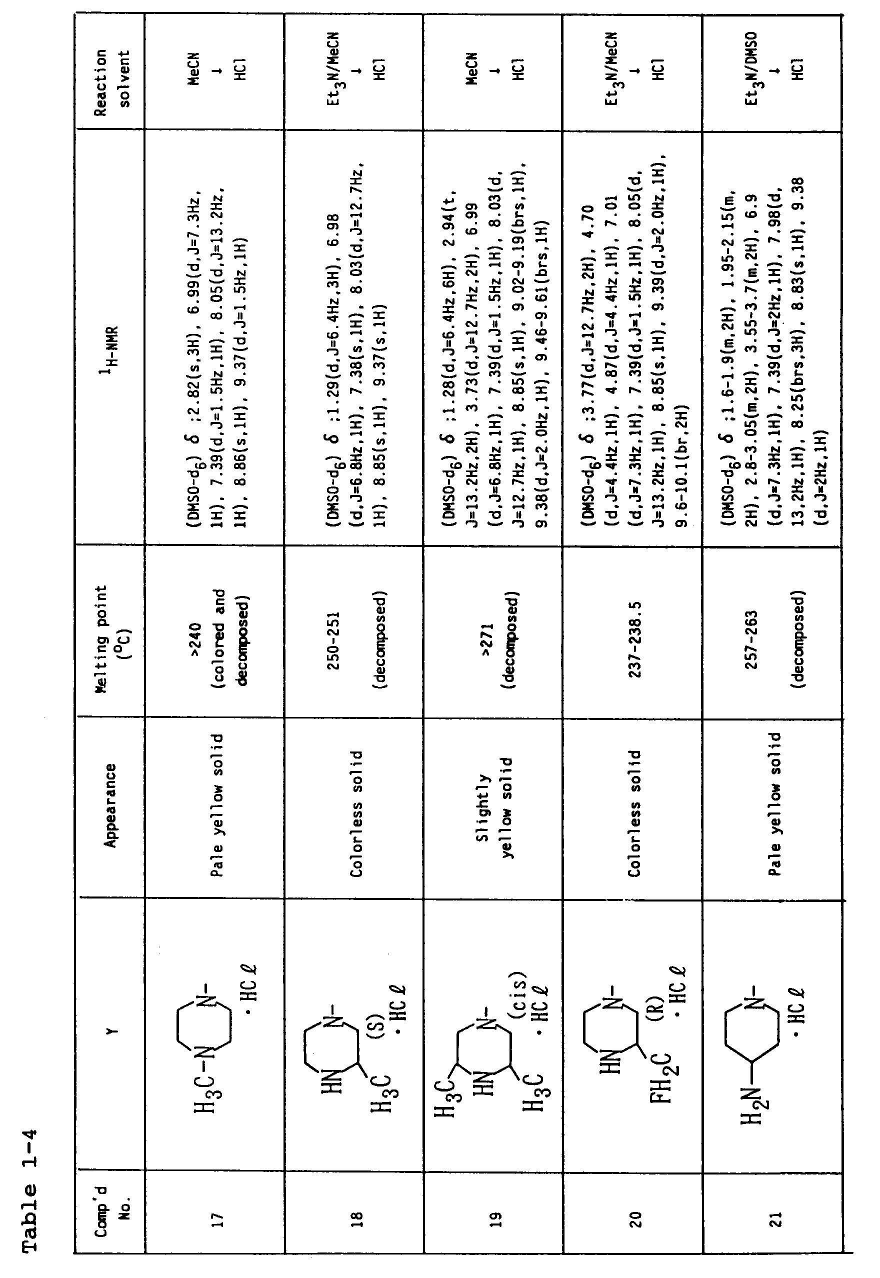

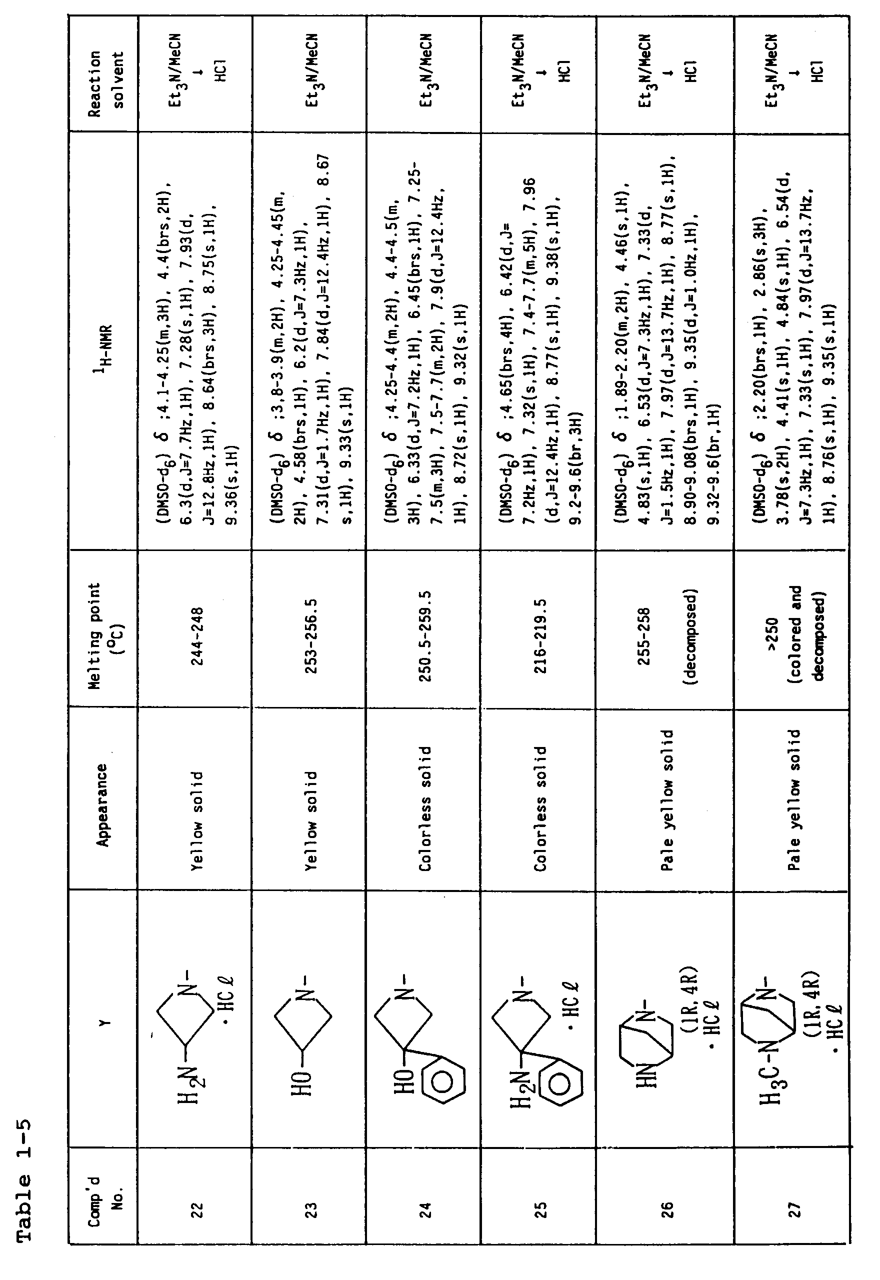

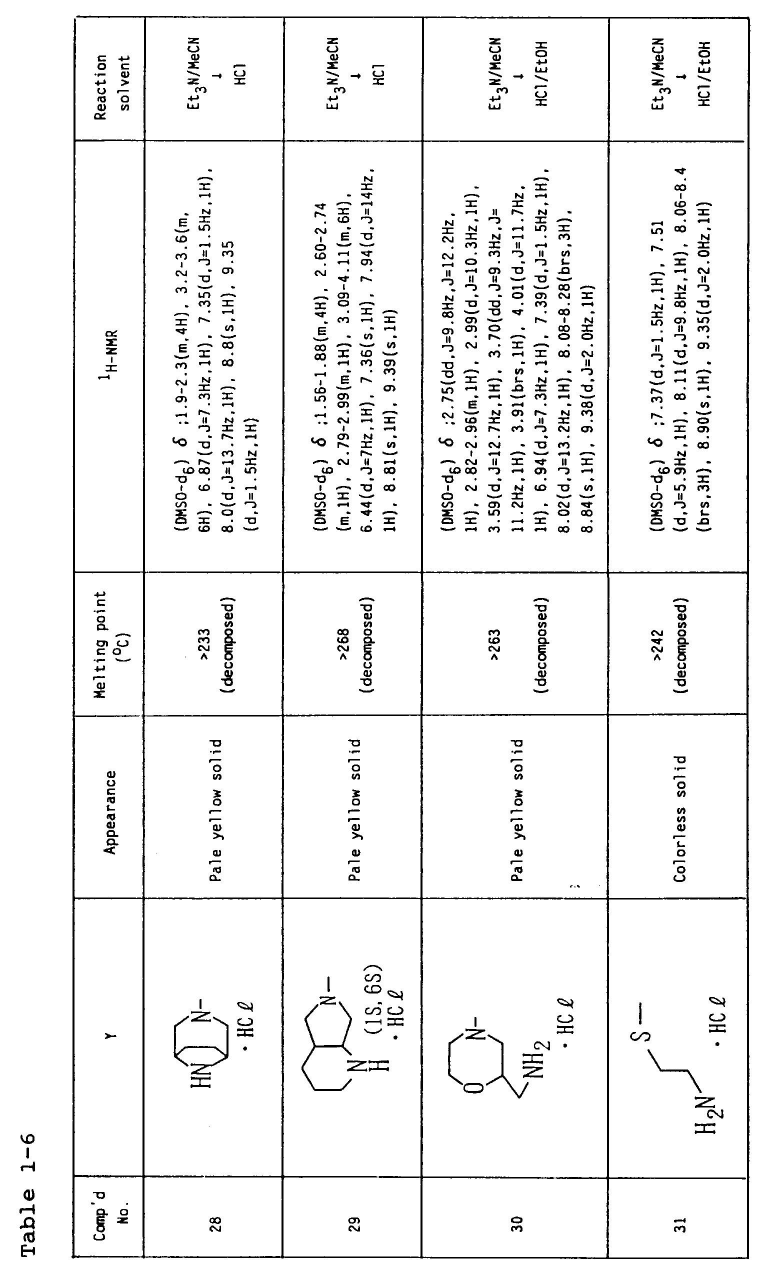

### Example 6

### Synthesis of ethyl 2-(3,4,6-trifluoro-2-methylbenzoyl)-3-(isoxazol-3-ylamino)acrylate (Compound No. 32)

Ethyl 3,4,6-trifluoro-2-methylbenzoyl acetate (1.05 g), 0.90 g of ethyl orthoformate and 1.86 g of acetic anhydride were combined, followed by heating at 130°C for 3 hours. Excess ethyl orthoformate and acetic anhydride were distilled off. The residue was then dissolved in 30 mℓ of chloroform. To the resulting solution, a solution of 376 mg of 3-aminoisoxazole in 30 mℓ of methanol was added, followed by stirring at room temperature for one hour. The solvent was distilled off. The residue was then purified using a silica gel column (chloroform), whereby 1.42 g of the title compound was obtained as a yellow oil.
- ¹H-NMR(CDCℓ₃)δ;: 0.97(t,J=7Hz) and 1.12(t,J=7Hz) for 3H, 2.21(d,J=2.4Hz) and 2.23(d,J=2.4Hz) for 3H, 3.99-4.16(m,2H), 6.39(t,J=1.5Hz,1H), 6.69-6.83(m,1H), 8.37(d,J=2.0Hz) and 8.41(d,J=2.0Hz) for 1H, 8.52(d,J=13.2Hz) and 8.68(d,J=13.2Hz) for 1H

### Example 7

### Synthesis of ethyl 6,7-difluoro-5-methyl-1-(isoxazol-3-yl)-1,4-dihydro-4-oxoquinoline-3- carboxylate (Compound No. 33)

In 25 mℓ of dimethylformamide, 1.22 g of Compound No. 32 were dissolved. To the resulting solution, 525 mg of potassium carbonate were added, followed by stirring at 90°C for 15 minutes. An aqueous solution of citric acid was added to the reaction mixture to neutralize the same. The solvent was distilled off. The residue was suspended in water, followed by collection through filtration. The solid so obtained was washed with water, ethanol and diethyl ether, whereby 940 mg of the title compound was obtained as a colorless solid.
- Melting point:: 200-202°C
- ¹H-NMR(CDCℓ₃)δ;: 1.38(t,J=7Hz,3H), 2.89(d,J=2.9Hz,3H), 4.38(q,J=7Hz,2H), 6.71(d,J=2.0Hz,1H), 6.98(dd,J=6.3Hz,10.3Hz,1H), 8.42(s,1H), 8.76(d,J=1.5Hz,1H)

### Example 8

### Synthesis of 6,7-difluoro-1-(isoxazol-3-yl)-5-methyl-1,4-dihydro-4-oxoquinoline-3-carboxylic acid (Compound No. 34)

To 940 mg of Compound No. 33, 30 mℓ of acetic acid and 15 mℓ of 6N hydrochloric acid were added, followed by stirring at 110°C for one hour. After the reaction mixture was allowed to cool down, water was added to cause further precipitation. The precipitate so obtained was collected by filtration, followed by washing with water, ethanol and diethyl ether, whereby 828 mg of the title compound were obtained as colorless needle crystals.
- Melting point:: 225-228°C
- ¹H-NMR(DMSO-d₆)δ;: 2.85(d,J=2.9Hz,3H), 7.23(d,J=2.0Hz,1H), 7.47(dd,J=7Hz,11.2Hz,1H), 8.87(s,1H), 9.36(d,J=1.5Hz,1H)

### Example 9

In a similar manner to Example 4, Compounds Nos. 35-46 shown in Tables 2-1 to 2-3 were synthesized using Compound No. 34.

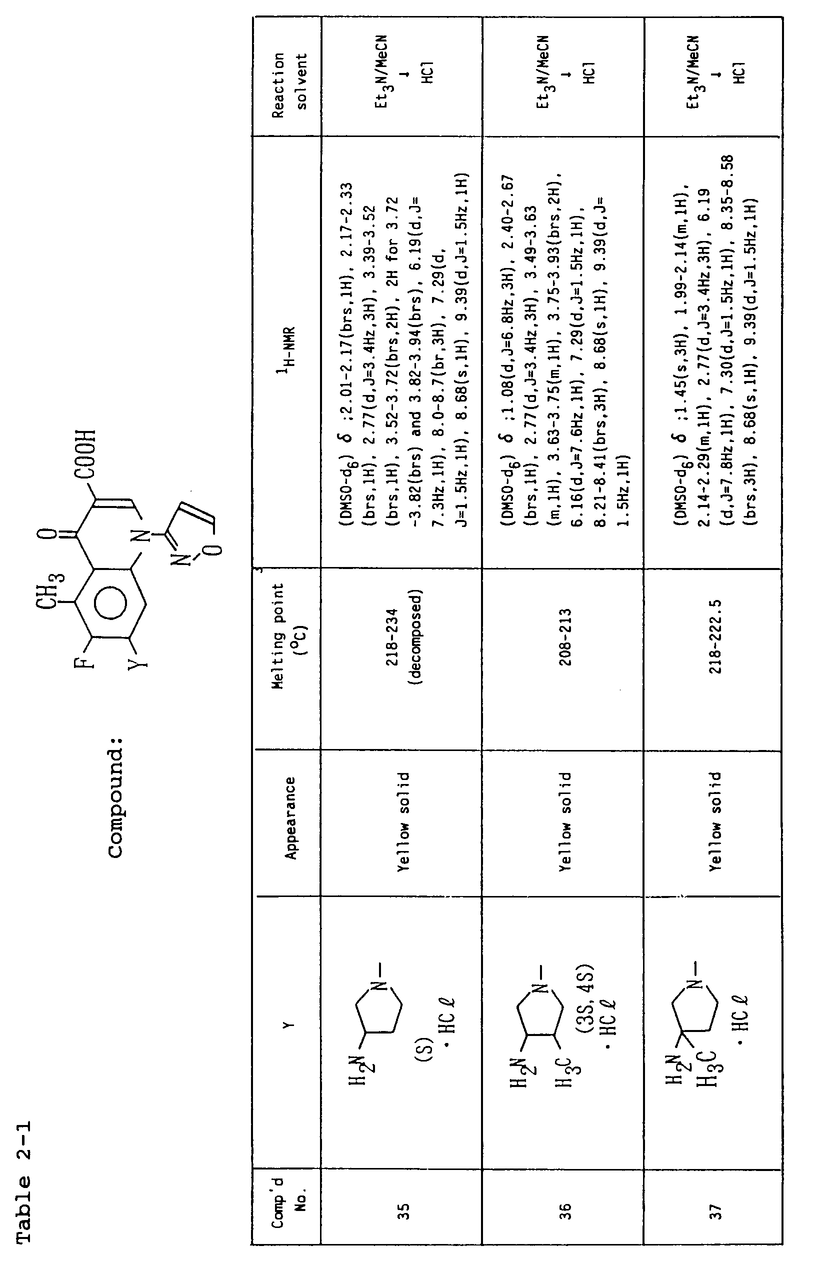

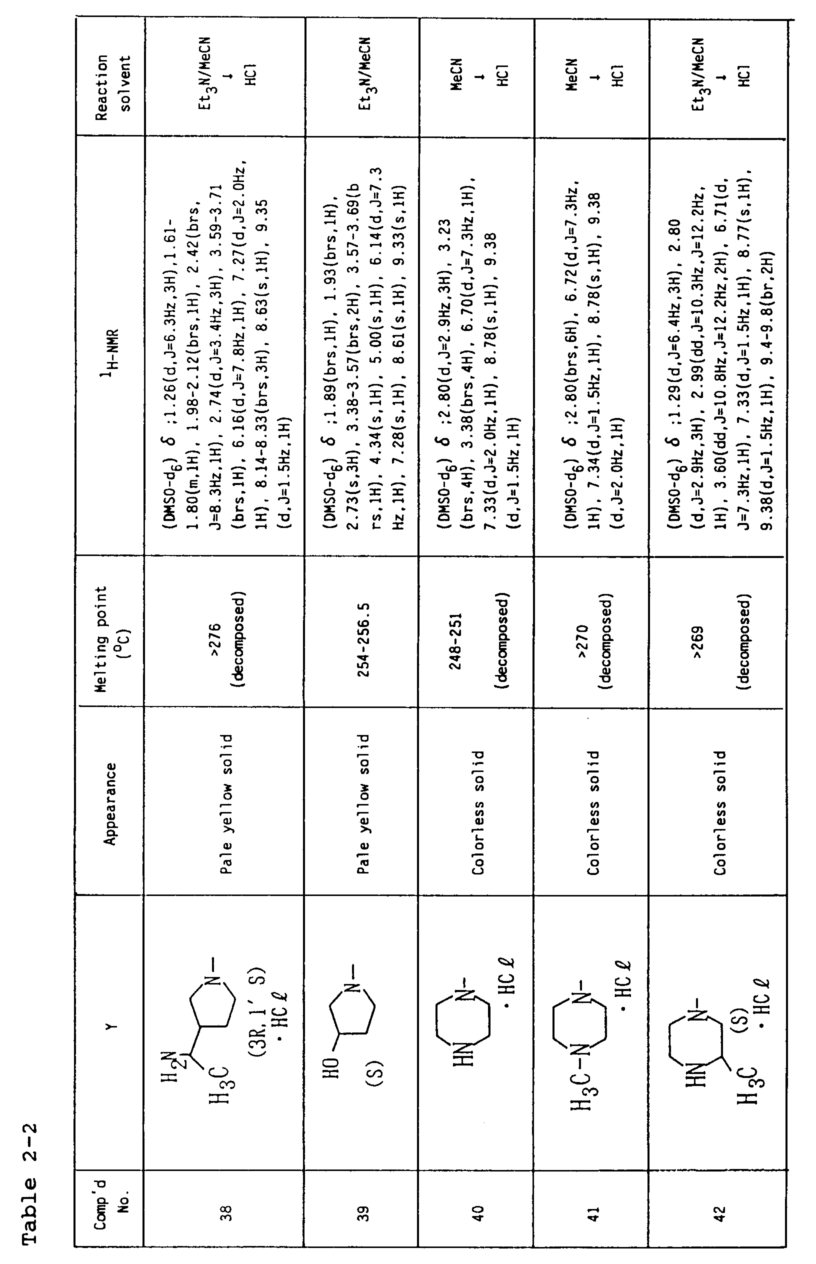

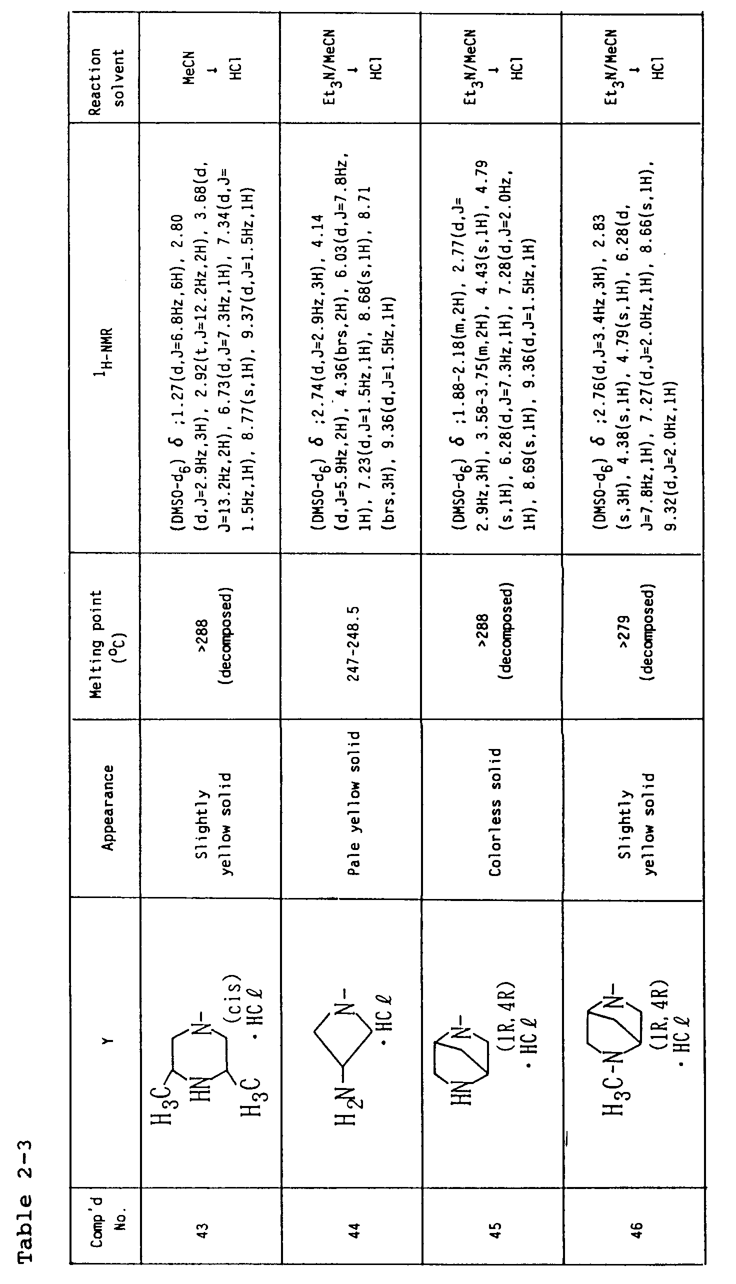

### Example 10

### Synthesis of ethyl 5-chloro-6,7-difluoro-1-(isoxazol-3-yl)-1,4-dihydro-4-oxoquinoline-3-carboxylate (Compound No. 47)

Ethyl 3,4,6-trifluoro-2-chlorobenzoylacetate (0.63 g), 0.63 mℓ of ethyl orthoformate and 0.7 mℓ of acetic anhydride were combined, followed by stirring at 130°C for one hour. Excess ethyl orthoformate and acetic anhydride were distilled off. To the residue, 5 mℓ of chloroform and 190 mg of 3-aminoisoxazole were added, followed by stirring at room temperature for 2 hours. The solvent was distilled off. To the residue, 20 mℓ of hexane were added. The solid so obtained (0.84 g as a yellow solid) was collected by filtration and then dissolved in 5 mℓ of dimethylformamide. To the resulting solution, 311 mg of anhydrous potassium carbonate were added, followed by stirring at 100°C for 10 minutes. The solvent was distilled off under reduced pressure. To the residue, 20 mℓ of 5% aqueous citric acid solution were added. The resulting mixture was extracted with 80 mℓ of chloroform. The extract was dried (MgSO₄), followed by the removal of the solvent through distillation, whereby 580 mg of the title compound were obtained as yellow powder.
- Melting point:: 179-184°C
- ¹H-NMR(CDCℓ₃)δ;: 1.37(t,J=7Hz,3H), 4.35(q,J=7Hz,2H), 6.85(d,J=1.7Hz,1H), 7.11(dd,J=10.7Hz,J=6.8Hz,1H), 8.39(s,1H), 8.77(d,J=1.7Hz,1H)

### Example 11

### Synthesis of 5-chloro-6,7-difluoro-1-(isoxazol-3-yl)-1,4-dihydro-4-oxoquinoline-3-carboxylic acid (Compound No. 48)

In 3 mℓ of 6N hydrochloric acid and 5 mℓ of acetic acid, 550 mg of Compound No. 47 were dissolved, followed by heating under reflux for one hour. After cooling, the reaction mixture was added with 20 mℓ of water. The solid so obtained was collected by filtration and then washed with water, ethanol and diethyl ether, whereby 470 mg of the title compound was obtained as a pale yellow solid.
- Melting point:: 228-233.5°C
- ¹H-NMR(DMSO-d₆)δ;: 7.2(d,J=1.7Hz,1H), 7.67(dd,J=11Hz,J=6.8Hz,1H), 8.88(s,1H), 9.37(d,J=1.7Hz,1H)

### Example 12

In a similar manner to Example 4, Compounds Nos. 49-52 shown in Table 3 were synthesized using Compound No. 48.

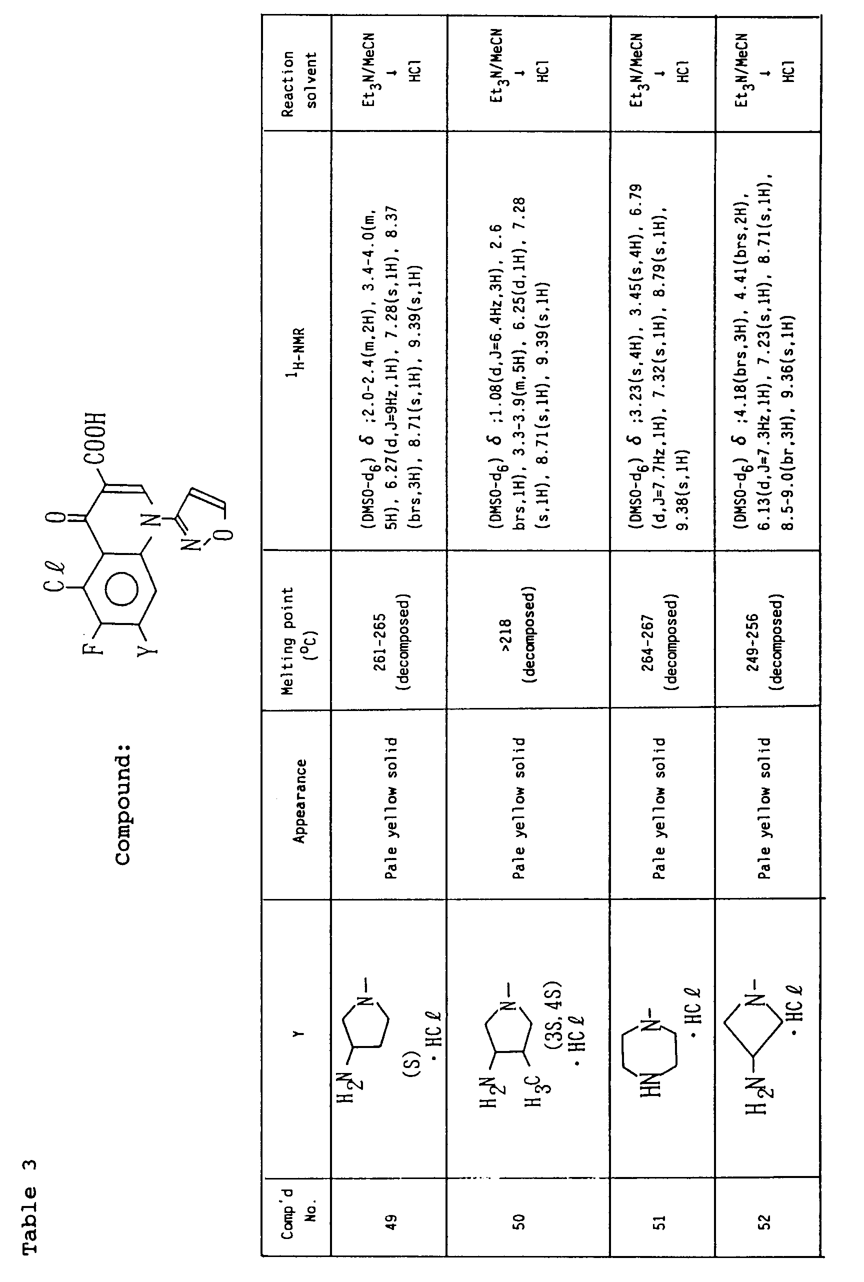

### Example 13

### Synthesis of ethyl 6,7-difluoro-5-nitro-1-(isoxozol-3-yl)-1,4-dihydro-4-oxoquinoline-3-carboxylate (Compound No. 53)

Ethyl 2-nitro-3,4,6-trifluorobenzoylacetate (7 g), 6.3 mℓ of ethyl orthoformate and 7.1 mℓ of acetic anhydride were combined, followed by stirring at 130°C for 2 hours. Excess ethyl orthoformate and acetic anhydride were distilled off. To the residue, 30 mℓ of chloroform, 2.1 g of 3-aminoisoxazole and 2.4g of triethylamine were added, followed by stirring at 50°C for 10 hours. The reaction mixture was washed with a 5% aqueous solution of citric acid and dried over anhydrous magnesium sulfate. The solvent was distilled off. The residue was suspended in ethanol, followed by collection through filtration, whereby 6.5 g of the title compound was obtained as a pale yellow solid.
- Melting point:: 193.5-195.5°C
- ¹H-NMR(CDCℓ₃)δ;: 1.34(t,J=7Hz,3H), 4.3(q,J=7Hz,2H), 7.0(d,J=1.7Hz,1H), 7.45(dd,J=10.7Hz,J=6.4Hz,1H), 8.51(s,1H), 8.8(d,J=1.7Hz,1H)

### Example 14

### Synthesis of 6,7-difluoro-5-nitro-1-(isoxazol-3-yl)-1,4-dihydro-4-oxoquinoline-3-carboxylic acid (Compound No. 54)

In 5 mℓ of acetic acid and 5 mℓ of 6N hydrochloric acid, 500 mg of Compound No. 53 were stirred at 100°C for 2 hours. After being cooled down, the reaction mixture was added with 30 mℓ of water. The solid so obtained was collected by filtration, whereby 410 mg of the title compound was obtained as a colorless solid.
- Melting point:: 243-248.5°C
- ¹H-NMR(DMSO-d₆)δ;: 7.21(d,J=1.7Hz,1H), 8.0(dd,J=11.5Hz,J=6.4Hz,1H), 8.89(s,1H), 9.36(d,J=1.7Hz,1H)

### Example 15

### Synthesis of ethyl 5-amino-6,7-difluoro-1-(isoxazol-3-yl)-1,4-dihydro-4-oxoquinoline-3-carboxylate (Compound No. 55)

In 160 mℓ of chloroform, 3 g of Compound No. 53 were dissolved. To the resulting solution, 10 drops of acetic acid and 300 mg of palladium carbon (10%) were added, followed by hydrogenation at room temperature for 30 hours. The catalyst was filtered out and the solvent was distilled off. The residue was suspended in isopropyl ether, followed by collection through filtration, whereby 2.5 g of the title compound was obtained as a pale yellow solid.
- Melting point:: 197-198.5°C
- ¹H-NMR(CDCℓ₃)δ;: 1.37(t,J=7Hz,3H), 4.37(q,J=7Hz,2H), 6.15(dd,J=11.5Hz,J=6Hz,1H), 6.7(d,J=1.7Hz,1H), 8.38(s,1H), 8.7(d,J=1.7Hz,1H)

### Example 16

### Synthesis of 5-amino-6,7-difluoro-1-(isoxazol-3-yl)-1,4-dihydro-4-oxoquinoline-3-carboxylic acid (Compound No. 56)

In 20 mℓ of acetic acid and 20 mℓ of 6N hydrochloric acid, 1.8 g of Compound No. 55 were stirred at 100°C for 2 hours, followed by the removal of the solvent through distillation under reduced pressure. To the residue, 100 mℓ of water was added, whereby 1.4 g of the title compound was collected by filtration as a pale yellow solid.
- Melting point:: 300°C or higher
- ¹H-NMR(DMSO-d₆)δ;: 6.38(dd,J=11.8Hz,J=6Hz,1H), 7.18(d,J=1.7Hz,1H), 8.0(brs,2H), 8.71(s,1H), 9.3(d,J=1.7Hz,1H)

### Example 17

In a similar manner to Example 4, Compounds Nos. 57-62 shown in Tables 4-1 to 4-2 were synthesized using Compound No. 56.

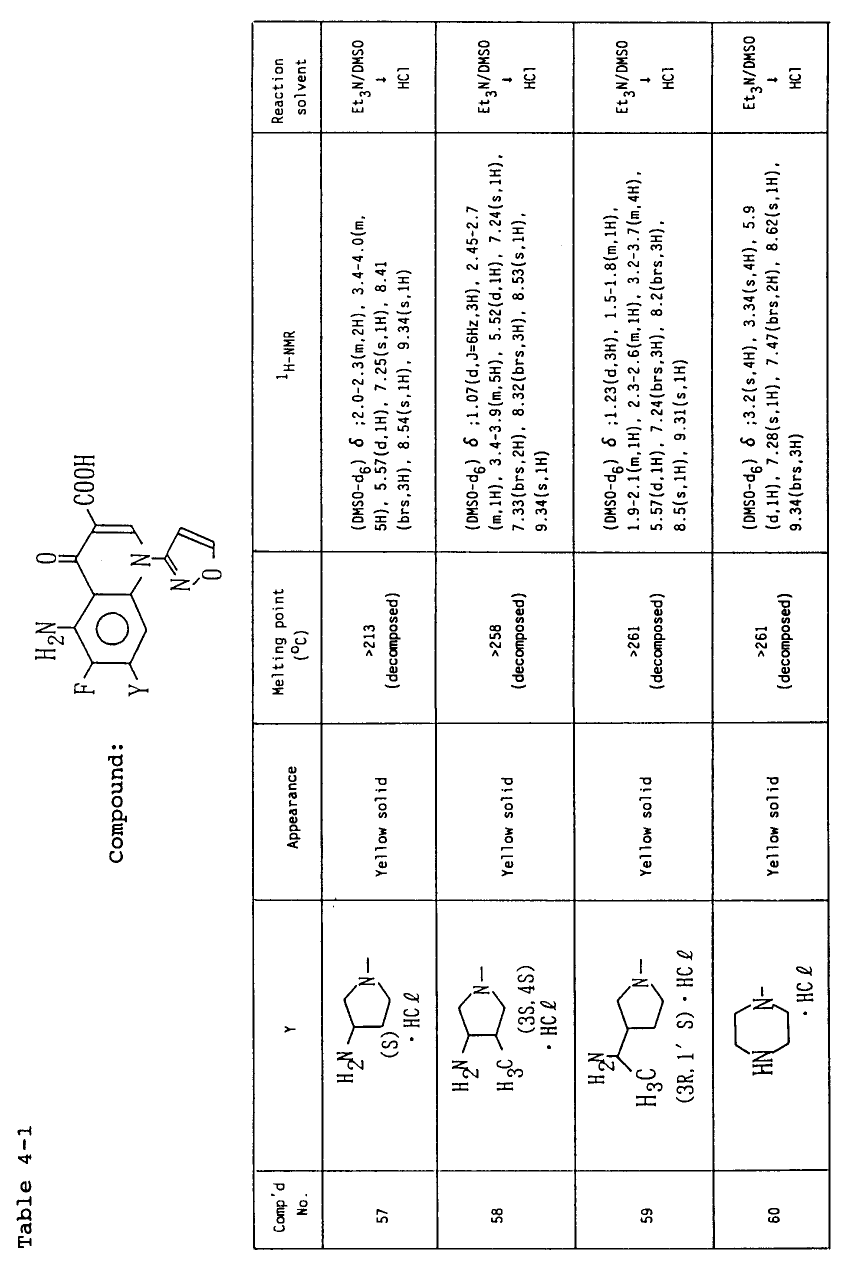

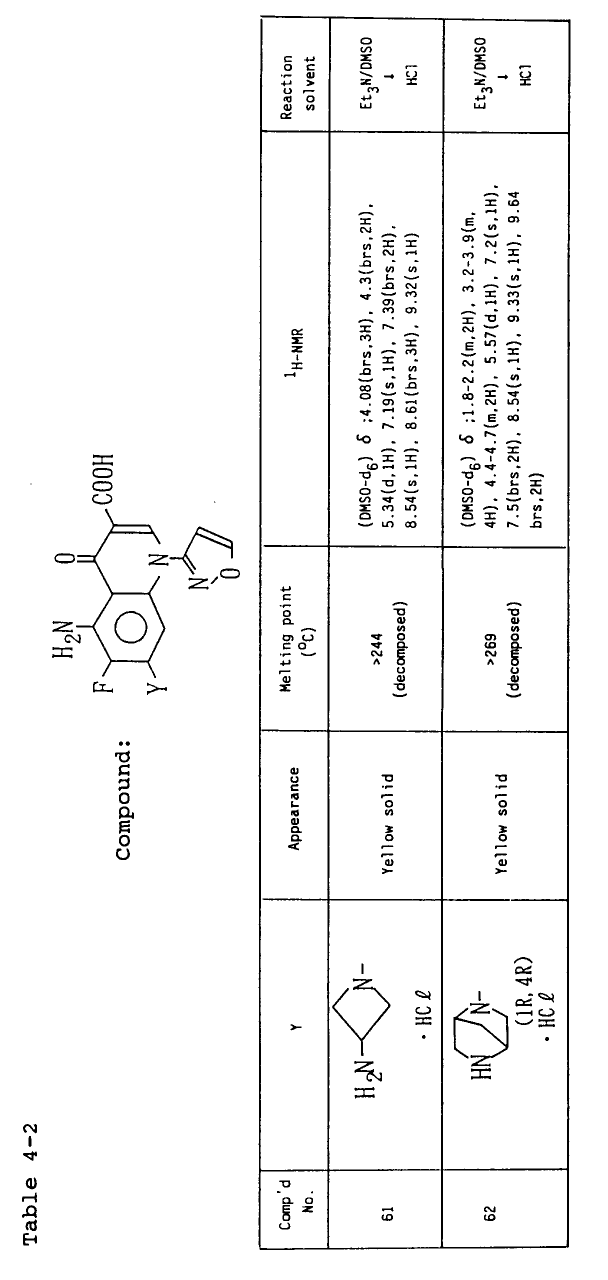

### Example 18

### Synthesis of ethyl 2-(2,3,4,5-tetrafluorobenzoyl)-3-(isoxazol-3-ylamino)acrylate (Compound No. 63)

Ethyl 2,3,4,5-tetrafluorobenzoylacetate (1.00 g), 0.84 g of ethyl orthoformate and 1.74 g of acetic anhydride were combined, followed by heating at 130°C for 3 hours. Excess ethyl orthoformate and acetic anhydride were distilled off. The residue was dissolved in 30 mℓ of chloroform. To the resulting solution, a solution of 320 mg of 3-aminoisoxazole in 20 mℓ of methanol was added, followed by stirring at room temperature for 2 hours. The solvent was distilled off. The residue was purified by using a silica gel column (hexane:chloroform = 1:2), whereby 1.28 g of the title compound was obtained as a yellow oil.
- ¹H-NMR(CDCℓ₃)δ;: 1.05(t,J=7Hz) and 1.19(t,J=7Hz) for 3H, 4.07-4.24(m,2H), 6.36(d,J=2.0Hz) and 6.38(d,J=1.5Hz) for 1H,, 7.01-7.16(m) and 7.19-7.32(m) for 1H, 8.36(d,J=13.2Hz) and 8.62(d,J=13.2Hz) for 1H, 8.37(d,J=2.0Hz) and 8.40(d,J=2.0Hz) for 1H.

### Example 19

### Synthesis of ethyl 6,7,8-trifluoro-1-(isoxazol-3-yl)-1,4-dihydro-4-oxoquinoline-3-carboxylate (Compound No. 64)

In 60 mℓ of tetrahydrofuran, 1.19 g of Compound No. 63 were dissolved. To the resulting solution, 162 mg of sodium hydride were added, followed by stirring at room temperature for 3 hours. An aqueous solution of citric acid was added to the reaction mixture to neutralize the same. The solvent was then distilled off. To the residue, a 5% aqueous solution of citric acid was added, followed by extraction with chloroform. After the extract was dried over sodium sulfate, the solvent was distilled off. The residue was purified using a silica gel column (chloroform), whereby 0.78 g of the title compound was obtained as a colorless solid.
- Melting point:: 210-214°C
- ¹H-NMR(CDCℓ₃)δ;: 1.39(t,J=7Hz,3H), 4.39(q,J=7Hz,2H), 6.69(t,J=1.5Hz,1H), 8.08-8.19(m,1H), 8.17(d,J=2.5Hz,1H), 8.43(s,1H), 8.66(d,J=2.0Hz,1H)

### Example 20

### Synthesis of 6,7,8-trifluoro-1-(isoxazol-3-yl)-1,4-dihydro-4-oxoquinoline-3-carboxylic acid (Compound No. 65)

To 660 mg of Compound No. 64, 25 mℓ of acetic acid and 15 mℓ of 6N hydrochloric acid were added, followed by stirring at 110°C for one hour. After being allowed to cool down, the reaction mixture was added with water. The solid so obtained was collected by filtration and washed with water, ethanol and diethyl ether, whereby 596 mg of the title compound was obtained as a pink solid.
- Melting point:: 215-217°C
- ¹H-NMR(DMSO-d₆)δ;: 7.21(t,J=1.0Hz,1H), 8.15-8.28(m,1H), 8.82(s,1H), 9.26(d,J=1.5Hz,1H)

### Example 21

In a similar manner to Example 4, Compounds Nos. 66-68 shown in Table 5 were synthesized using Compound No. 65.

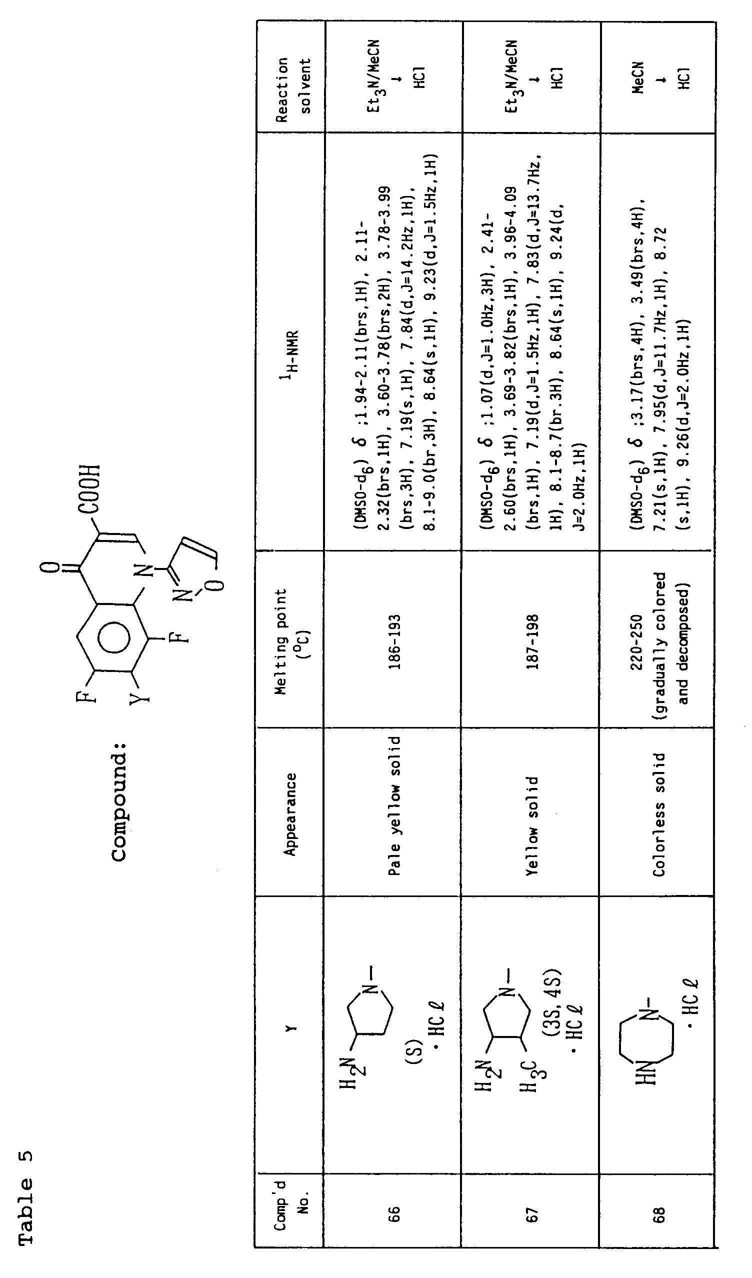

### Example 22

### Synthesis of ethyl 5-benzyloxy-6,7,8-trifluoro-1-(isoxazol-3-yl)-1,4-dihydro-4-oxoquinoline-3-carboxylate (Compound No. 69)

Ethyl 2-benzyloxy-3,4,5,6-tetrafluorobenzoylacetate (3.7 g), 2.5 mℓ of ethyl orthoformate and 2.8 mℓ of acetic anhydride were combined, followed by stirring at 130°C for one hour. Excess ethyl orthoformate and acetic anhydride were distilled off. To the residue, 20 mℓ of chloroform and 840 mg of 3-aminoisoxazole were added, followed by stirring at room temperature for 3 hours. The solvent was distilled off. The residue was dissolved in 20 mℓ of dimethylformamide. To the resulting solution, 1.38 g of potassium carbonate were added, followed by stirring at 100°C for 15 minutes. The solvent was distilled off and to the residue, 50 mℓ of 5% aqueous citric acid solution were added. The resulting mixture was extracted with 10 mℓ of chloroform. The solvent was distilled off. The residue was purified using a silica gel column (chloroform:ethyl acetate = 4:1), whereby 2.1 g of the title compound was obtained as pale yellow powder.
- Melting point:: 169-174.5°C
- ¹H-NMR(CDCℓ₃)δ;: 1.37(t,J=7Hz,3H), 4.36(q,J=7Hz,2H), 5.24(s,2H), 6.63(s,1H), 7.3-7.45(m,3H), 7.55-7.65(m,2H), 8.3(s,1H), 8.58(s,1H)

### Example 23

### Synthesis of ethyl 5-hydroxy-6,7,8-trifluoro-1-(isoxazol-3-yl)-1,4-dihydro-4-oxoquinoline-3-carboxylate (Compound No. 70)

In 2 mℓ of chloroform, 150 mg of Compound No. 69 were dissolved. To the resulting solution, 2 mℓ of 4N hydrochloric acid/dioxane were added, followed by stirring at 50°C for 3 hours. The solvent was distilled off. To the residue, 3 mℓ of diethyl ether were added. The solid so obtained was collected by filtration, whereby 105 mg of the title compound was obtained as a yellow solid.
- Melting point:: 180.5-187°C
- ¹H-NMR(CDCℓ₃)δ;: 1.39(t,J=7Hz,3H), 4.39(q,J=7Hz,2H), 6.67(d,J=1.7Hz,1H), 8.42(s,1H), 8.65(d,J=1.7Hz,1H)

### Example 24

### Synthesis of 5-hydroxy-6,7,8-trifluoro-1-(isoxazol-3-yl)-1,4-dihydro-4-oxoquinoline-3-carboxylic acid (Compound No. 71)

Process A In a mixed solution of acetic acid and 6N hydrochloric acid, 90 mg of Compound No. 69 were hydrolyzed at 100°C. After being cooled down, the hydrolyzate was added with water. The solid so obtained was collected by filtration, whereby 70 mg of the title compound was obtained as a pale yellow solid.

Process B In a mixed solution of 5 mℓ of acetic acid and 6 mℓ of 6N hydrochloric acid, 0.97 g of Compound No. 68 was stirred at 100°C for 3 hours. After being cooled down, the reaction mixture was added with 30 mℓ of water. The solid so obtained was collected by filtration, whereby 570 mg of the title compound was obtained as a pale yellow solid.
- Melting point:: 235.5-239°C
- ¹H-NMR(DMSO-d₆)δ;: 7.16(s,1H), 8.68(s,1H), 9.23(s,1H)

### Example 25

In a similar manner to Example 4, Compounds Nos. 72-74 shown in Table 6 were synthesized using Compound No. 71.

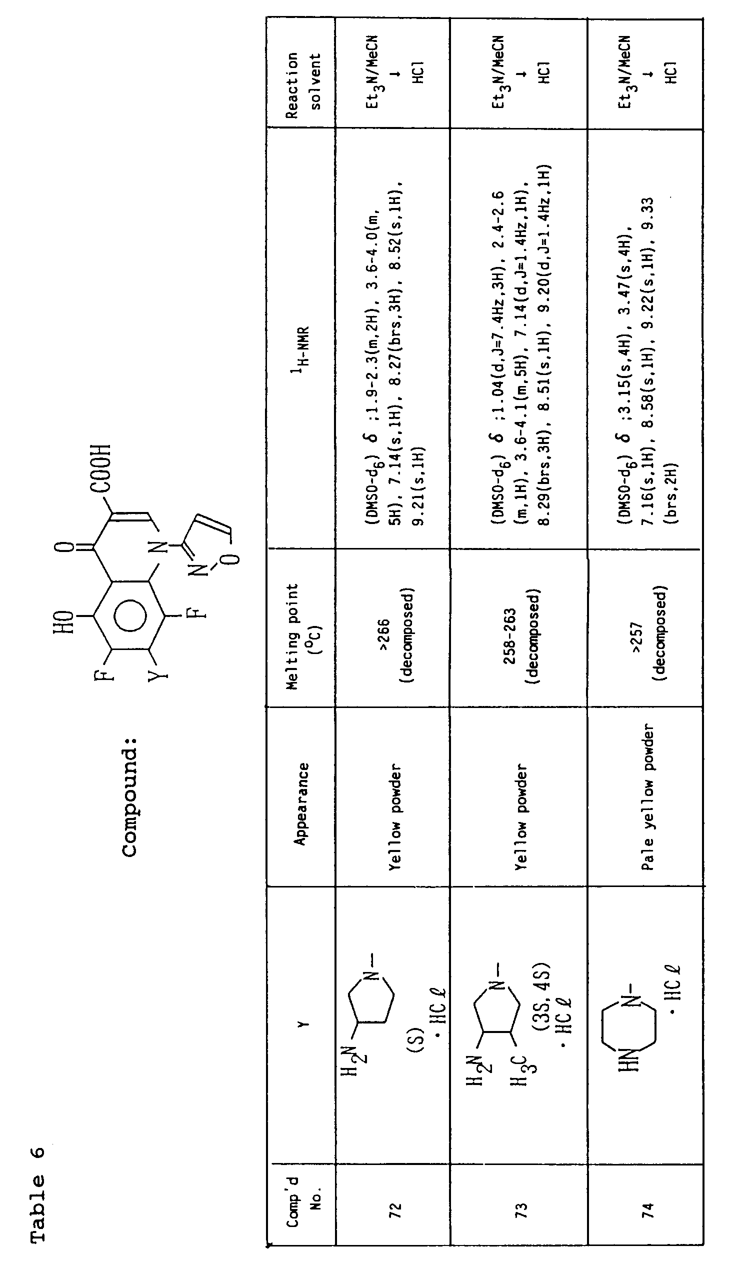

### Example 26

### Synthesis of ethyl 8-chloro-6,7-difluoro-1-(isoxazol-3-yl)-1,4-dihydro-4-oxoquinoline-3-carboxylate (Compound No. 75)

Ethyl 2,4,5-trifluoro-3-chlorobenzoylacetate (1.4 g), 1.3 mℓ of ethyl orthoformate and 1.4 mℓ of acetic anhydride were combined, followed by stirring at 130°C for 2 hours. Excess ethyl orthoformate and acetic anhydride were distilled off. To the residue, 10 mℓ of chloroform and 420 mg of 3-aminoisoxazole were added, followed by stirring at room temperature for 6 hours. The solvent was distilled off. To the residue, 330 mℓ of hexane were added. The solid so obtained (1.7 g as yellow powder) were collected by filtration. This solid (1.7 g) was then dissolved in 5 mℓ of dimethylformamide. To the resulting solution, 690 mg of potassium carbonate were added, followed by stirring at 100°C for 10 minutes. After the solvent was distilled off, 10 mℓ of 5% aqueous citric acid solution was added to the residue. The solid so obtained was collected by filtration and then washed with water, ethanol and isopropyl ether, each 10 mℓ, whereby 1.2 g of the title compound was obtained as a pale yellow solid.
- Melting point:: 188-191.5°C
- ¹H-NMR(CDCℓ₃)δ;: 1.37(t,J=7Hz,3H), 4.35(q,J=7Hz,2H), 6.85(d,J=1.7Hz,1H), 7.1(dd,J=10.7Hz,J=6.8Hz,1H), 8.4(s,1H), 88.77(d,J=1.7Hz,1H)

### Example 27

### Synthesis of 8-chloro-6,7-difluoro-1-(isoxazol-3-yl)-1,4-dihydro-4-oxoquinoline-3-carboxylic acid (Compound No. 76)

In a mixed solution of 3 mℓ of acetic acid and 6 mℓ of 6N hydrochloric acid, 1.2 g of Compound No. 75 were stirred at 100°C for 1.5 hours. After being cooled down, the reaction mixture was added with 30 mℓ of water. The solid so obtained was collected by filtration and then washed with water, ethanol and diethyl ether, whereby 0.99 of the title compound was obtained as a colorless solid.
- Melting point:: 220-221.5°C
- ¹H-NMR(DMSO-d₆)δ;: 7.2(d,J=1.7Hz,1H), 8.36(dd,J=10Hz,J=8.6Hz,1H), 8.74(s,1H), 9.26(d,J=1.7Hz,1H)

### Example 28

### Synthesis of 8-chloro-6-fluoro-7-(pyrrolidin-1-yl)-1-(isoxazol-3-yl)-1,4-dihydro-4-oxoquinoline-3-carboxylic acid (Compound No. 77)

In 2 mℓ of acetonitrile and 1 mℓ of dimethyl sulfoxide, 80 mg of Compound No. 76 were dissolved. To the resulting solution, 17 mg of pyrrolidine and 50 mg of triethylamine were added, followed by stirring at 60°C for 30 minutes. After the reaction mixture was cooled down, the solid so obtained was collected by filtration. The solid was then washed with diethyl ether, whereby 40 mg of the title compound was obtained as a colorless solid.
- Melting point:: 249-253°C
- ¹H-NMR(CDCℓ₃)δ;: 1.8-2.0(m,4H), 3.45-3.6(m,4H), 6.55(d,J=1.7Hz,1H), 7.93(d,J=13Hz,1H), 8.52(d,J=1.7Hz,1H), 8.62(s,1H)

### Example 29

### Synthesis of ethyl 2-(2,4,5-trifluorobenzoyl)-3-(4-methylisoxazol-3-ylamino)acrylate (Compound No. 78)

Ethyl 2,4,5-trifluorobenzoylacetate (2.00 g), 1.81 g of ethyl orthoformate and 3.73 g of acetic anhydride were combined, followed by heating at 130°C for 3 hours. Excess ethyl orthoformate and acetic anhydride were distilled off. The residue was dissolved in 30 mℓ of chloroform. To the resulting solution, a solution of 797 mg of 3-amino-4-methylisoxazole in 20 mℓ of chloroform were added, followed by stirring at room temperature for 2 days. The solvent was distilled off. The residue was purified using a silica gel column (hexane:chloroform = 2:1), whereby 2.55 g of the title compound was obtained as a yellow oil.

### [0118]

- Melting point:: 72-74.5°C
- ¹H-NMR(CDCℓ₃)δ;: 1.04(t,J=7Hz) and 1.20(t,J=7Hz) for 3H, 2.09(d,J=1.0Hz) and 2.11(d,J=1.0Hz) for 3H, 4.09-4.25(m,2H), 6.86-7.02(m,1H), 7.33(dd,J=3Hz,9Hz) and 7.50(dd,J=3Hz,J=9Hz) for 1H, 8.12(d,J=1.0Hz) and 8.16(d,J=1.0Hz) for 1H, 8.52(d,J=13.2Hz) and 8.78(d,J=12.7Hz) for 1H

### Example 30

### Synthesis of ethyl 6,7-difluoro-1-(4-methylisoxazol-3-yl)-1,4-dihydro-4-oxoquinoline-3-carboxylate (Compound No. 79)

In 100 mℓ of tetrahydrofuran, 2.35 g of Compound No. 78 were dissolved. To the resulting solution, 319 mg of sodium hydride were added, followed by stirring at room temperature for one hour. An aqueous solution of citric acid was added to the reaction mixture to neutralize the same. The solvent was then distilled off. To the residue, a 5% aqueous solution of citric acid was added, followed by extraction with chloroform. The extract was dried over sodium sulfate and then the solvent was distilled off. The residue was purified using a silica gel column (chloroform:ethyl acetate = 10:1), whereby 1.63 g of the title compound was obtained as a colorless solid.
- Melting point:: 223-226°C
- ¹H-NMR(CDCℓ₃)δ;: 1.40(t,J=7Hz,3H), 2.02(s,3H), 4.40(g,J=7Hz,2H), 6.89(dd,J=5.9Hz,10.8Hz,1H), 8.31(dd,J=8.8Hz,10.3Hz,1H), 8.44(s,1H), 8.57(d,J=1.0Hz,1H)

### Example 31

### Synthesis of 6,7-difluoro-1-(4-methylisoxazol-3-yl)-1,4-dihydro-4-oxoquinoline-3-carboxylic acid (Compound No. 80)

To 1.60 g of Compound No. 79, 60 mℓ of acetic acid and 30 mℓ of 6N hydrochloric acid were added, followed by stirring at 110°C for one hour. After being allowed to cool down, the reaction mixture was added with water. The solid so obtained was collected by filtration and then washed with water, ethanol and diethyl ether, whereby 1.33 g of the title compound was obtained as a colorless solid.
- Melting point:: 242-246°C
- ¹H-NMR(DMSO-d₆)δ;: 1.90(s,3H), 7.55(dd,J=6.4Hz,11.2Hz,1H), 8.36(dd,J=8.30Hz,J=10.3Hz,1H), 8.98(s,1H), 9.17(d,J=1.0Hz,1H)

### Example 32

In a similar manner to Example 4, Compounds Nos. 81 and 82 shown in Table 7 were synthesized using Compound No. 80.

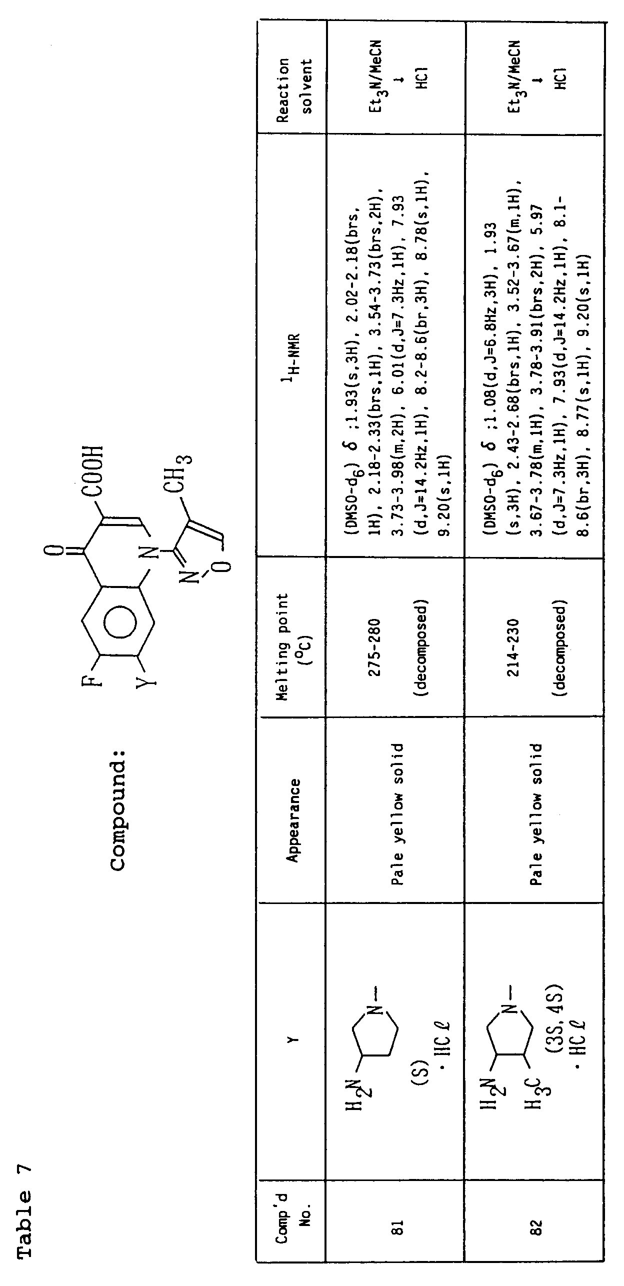

### Example 33

### Synthesis of ethyl 2-(2,4,5-trifluorobenzoyl)-3-(5-methylisoxazol-3-ylamino)acrylate (Compound No. 83)

Ethyl 2,4,5-trifluorobenzoylacetate (1.00 g), 0.91 g of ethyl orthoformate and 1.87 g of acetic anhydride were combined, followed by heating at 130°C for 3 hours. Excess ethyl orthoformate and acetic anhydride were distilled off. The residue was dissolved in 20 mℓ of chloroform. To the resulting solution, a solution of 400 mg of 3-amino-5-methylisoxazole in 15 mℓ of methanol was added, followed by stirring overnight at room temperature. The solvent was distilled off and the residue was purified using a silica gel column (hexane:chloroform = 1:2), whereby 1.02 g of the title compound was obtained as a colorless solid.
- Melting point:: 87-87.5°C
- ¹H-NMR(CDCℓ₃)δ;: 1.02(t,J=7Hz) and 1.16(t,J=7Hz) for 3H, 2.44(s) and 2.45(s) for 3H, 4.08-4.19(m,2H), 6.00(s) and 6.01(s) for 1H, 6.84-6.97(m,1H), 7.28-7.38(m) and 7.42-7.53(m) for 1H, 8.26(d,J=13.2Hz) and 8.52(d,J=13.2Hz) for 1H

### Example 34

### Synthesis of ethyl 6,7-difluoro-1-(5-methylisoxazol-3-yl)-1,4-dihydro-4-oxoquinoline-3-carboxylate (Compound No. 84)

In 20 mℓ of dimethylformamide, 990 mg of Compound No. 83 were dissolved. To the resulting solution, 425 mg of potassium carbonate were added, followed by stirring at 90°C for 10 minutes. An aqueous solution of citric acid was added to the reaction mixture to neutralize the same. The solvent was then distilled off. The residue was suspended in water, followed by collection through filtration. The solid so obtained was washed with water, ethanol and diethyl ether, whereby 804 mg of the title compound was obtained as a colorless solid.
- Melting point:: 173.5-175°C
- ¹H-NMR(CDCℓ₃)δ;: 1.40(t,J=7Hz,3H), 2.64(d,J=1.0Hz,3H), 4.39(q,J=7Hz,2H), 6.39(s,1H), 7.36(dd,J=6.3Hz,J=11.2Hz,1H), 8.27(dd,J=8.8Hz,J=10.3Hz,1H), 8.54(s,1H)

### Example 35

### Synthesis of 6,7-difluoro-1-(5-methylisoxazol-3-yl)-1,4-dihydro-4-oxoquinoline-3-carboxylic acid (Compound No. 85)

To 770 mg of Compound No. 84, 30 mℓ of acetic acid and 15 mℓ of 6N hydrochloric acid were added, followed by stirring at 110°C for one hour. After being allowed to cool down, the reaction mixture was added with water. The solid so obtained was collected by filtration and then washed with water, ethanol and diethyl ether, whereby 683 mg of the title compound was obtained as colorless needle crystals.
- Melting point:: 254-261°C
- ¹H-NMR(DMSO-d₆)δ;: 2.59(s,3H), 6.93(s,1H), 7.80(dd,J=6.8Hz,J=11.7Hz,1H), 8.33(dd,J=8.3Hz,J=10.3Hz,1H), 8.90(s,1H)

### Example 36

### Synthesis of 7-{3(S)-aminopyrrolidin-1-yl}-6-fluoro-1-(5-methylisoxazol-3-yl)-1,4-dihydro-4-oxoquinoline-3-carboxylic acid hydrochloride (Compound No. 86)

In 5 mℓ of acetonitrile, 50 mg of Compound No. 85 were dissolved. To the resulting mixture, 18 mg of 3(S)-aminopyrrolidine and 42 mg of triethylamine were added, followed by stirring at 80°C for one hour. The precipitate so obtained was collected by filtration, washed with ethanol, and the dissolved in 6N hydrochloric acid to obtain its hydrochloride. The solvent was distilled off. The residue was suspended in diethyl ether, followed by collection through filtration, whereby 59 mg of the title compound was obtained as a pale yellow solid.
- Melting point:: 247°C or higher (decomposed)
- ¹H-NMR(DMSO-d₆)δ;: 2.05-2.19(brs,1H), 2.19-2.38(brs,1H), 2.60(s,3H), 3.61-3.78(brs,2H), 3.78-4.01(m,2H), 6.50(d,J=7.3Hz,1H), 6.98(s,1H), 7.89(d,J=14.2Hz,1H), 8.2-8.5(brs,3H), 8.69(s,1H)

### Example 37

### Synthesis of ethyl 2-(2,4,5-trifluorobenzoyl)-3-(isoxazol-5-ylamino)acrylate (Compound No. 87)

Ethyl 2,4,5-trifluorobenzoylacetate (1.50 g), 1.36 g of ethyl orthoformate and 2.80 g of acetic anhydride were combined, followed by heating at 130°C for 3 hours. Excess ethyl orthoformate and acetic anhydride were distilled off. The residue was dissolved in 30 mℓ of chloroform. To the resulting solution, a solution of 512 mg of 5-aminoisoxazole in 30 mℓ of methanol was added, followed by stirring at room temperature for 3 hours. The solvent was distilled off. The residue was purified using a silica gel column (hexane:chloroform = 1:1), whereby 1.74 g of the title compound was obtained as a colorless solid.
- Melting point:: 103-107°C
- ¹H-NMR(CDCℓ₃)δ;: 1.03(t,J=7Hz) and 1.18(t,J=7Hz) for 3H, 4.08-4.23(m,2H), 5.76(d,J=2.0Hz) and 5.82(d,J=2.0Hz) for 1H, 6.85-7.00(m,1H), 7.29-7.40(m) and 7.46-7.58(m) for 1H, 8.18(d,J=12.7Hz) and 8.45(d,J=12.7Hz) for 1H, 8.19(d,J=1.5Hz) and 8.23(d,J=2.0Hz) for 1H

### Example 38

### Synthesis of ethyl 6,7-difluoro-1-(isoxazol-5-yl)-1,4-dihydro-4-oxoquinoline-3-carboxylate (Compound No. 88)

In 75 mℓ of tetrahydrofuran, 1.49 g of Compound No. 87 were dissolved. To the resulting solution, 215 mg of sodium hydride were added, followed by stirring at room temperature for one day. An aqueous solution of citric acid was added to the reaction mixture to neutralize the same. The solvent was then distilled off. To the residue, 5% citric acid was added. The resulting mixture was extracted with chloroform, followed by dehydration over a Glauber's salt. The solvent was then distilled off. The residue was purified using a silica gel column (chloroform), whereby 274 mg of the title compound was obtained as a pale yellow solid.
- Melting point:: 205-215°C (decomposed)
- ¹H-NMR(CDCℓ₃)δ;: 1.40(t,J=7Hz,3H), 4.41(q,J=7Hz,2H), 6.56(d,J=2.4Hz,1H), 7.07(dd,J=6.4Hz,J=10.7Hz,1H), 8.28(dd,J=8.8Hz,J=9.8Hz,1H), 8.47(s,1H), 8.56(s,1H)

### Example 39

### Synthesis of 6,7-difluoro-1-(isoxazol-5-yl)-1,4-dihydro-4-oxoquinoline-3-carboxylic acid (Compound No. 89)

To 250 mg of Compound No. 88, 10 mℓ of acetic acid and 5 mℓ of 6N hydrochloric acid were added, followed by stirring at 110°C for one hour. After being allowed to cool down, the reaction mixture was added with water. The solid so obtained was collected by filtration and then washed with water, ethanol and diethyl ether, whereby 198 mg of the title compound was obtained as a slightly yellow solid.
- Melting point:: 214-224°C (decomposed)
- ¹H-NMR(DMSO-d₆)δ;: 7.15(d,J=2.0Hz,1H), 7.58(dd,J=6.4Hz,J=11.2Hz,1H), 8.33(dd,J=8.8Hz,J=10.3Hz,1H), 8.98(s,1H), 8.99(s,1H)

### Example 40

### Synthesis of ethyl 2-(2,4,5-trifluorobenzoyl)-3-(3-methylisoxazol-5-ylamino)acrylate (Compound No. 90)

Ethyl 2,4,5-trifluorobenzoylacetate (1.00 g), 0.91 g of ethyl orthoformate and 1.87 g of acetic anhydride were combined, followed by heating at 130°C for 3 hours. Excess ethyl orthoformate and acetic anhydride were distilled off. The residue was dissolved in 20 mℓ of chloroform. To the resulting solution, a solution of 400 mg of 5-amino-3-methylisoxazole in 15 mℓ of methanol was added, followed by stirring at room temperature overnight. The solvent was then distilled off. The residue was suspended in hexane and a small amount of ethyl acetate, followed by collection through filtration, whereby 983 mg of the title compound were obtained as pale yellow needle crystals.
- Melting point:: 110-111°C
- ¹H-NMR(CDCℓ₃)δ;: 1.03(t,J=7Hz) and 1.18(t,J=7Hz) for 3H, 2.28(s) and 2.30(s) for 3H, 4.06-4.20(m,2H), 5.62(s) and 5.68(s) for 1H, 6.85-6.98(m,1H), 7.29-7.39(m) and 7.47-7.57(m) for 1H, 8.15(d,J=12.7Hz) and 8.42(d,J=12.7Hz) for 1H.

### Example 41

### Synthesis of ethyl 6,7-difluoro-1-(3-methylisoxazol-5-yl)-1,4-dihydro-4-oxoquinoline-3-carboxylate (Compound No. 91)

In 20 mℓ of dimethylformamide, 950 mg of Compound No. 90 were dissolved. To the resulting solution, 410 mg of potassium carbonate were added, followed by stirring at 90°C for 10 minutes. An aqueous solution of citric acid was added to the reaction mixture to neutralize the same. The solvent was then distilled off. The residue was suspended in water, followed by collection through filtration. The solid so obtained was washed with water, ethanol and diethyl ether, whereby 768 mg of the title compound was obtained as a pale yellow solid.
- Melting point:: 166-167°C
- ¹H-NMR(CDCℓ₃)δ;: 1.40(t,J=7Hz,3H), 2.49(s,3H), 4.40(q,J=7Hz,2H), 6.42(s,1H), 7.11(dd,J=6.4Hz,J=10.8Hz,1H), 8.25(dd,J=8.3Hz,J=10Hz,1H), 8.46(s,1H)

### Example 42

### Synthesis of 6,7-difluoro-1-(3-methylisoxazol-5-yl)-1,4-dihydro-4-oxoquinoline-3-carboxylic acid (Compound No. 92)

To 730 mg of Compound No. 91, 30 mℓ of acetic acid and 15 mℓ of 6N hydrochloric acid were added, followed by stirring at 110°C for one hour. After being allowed to cool down, the reaction mixture was added into water. The solid so formed was collected by filtration, followed by washing with water, ethanol and diethyl ether, whereby 642 mg of the title compound were obtained as colorless needle crystals.
- Melting point:: 248-250°C
- ¹H-NMR(DMSO-d₆)δ;: 2.40(s,3H), 7.01(s,1H), 7.63(dd,J=6.4Hz,J=11.2Hz,1H), 8.32(dd,J=8.3Hz,J=10Hz,1H), 8.94(s,1H)

### Example 43

### Synthesis of 7-{3(S)-aminopyrrolidin-1-yl}-6-fluoro-1-(3-methylisoxazol-5-yl)-1,4-dihydro-4-oxoquinoline-3-carboxylic acid hydrochloride (Compound No. 93)

In 55 mℓ of acetonitrile, 50 mg of Compound No. 92 were dissolved. To the resulting solution, 17 mg of 3(S)-aminopyrrolidine and 37 mg of triethylamine were added, followed by stirring at 80°C for one hour. The precipitate so obtained was collected by filtration, washed with ethanol and then dissolved in 6N hydrochloric acid to obtain its hydrochloride. The solvent was distilled off. The residue was suspended in diethyl ether, followed by collection through filtration, whereby 58 mg of the title compound was obtained as a pale yellow solid.
- Melting point:: 252°C or higher (decomposed)
- ¹H-NMR(DMSO-d₆)δ;: 2.05-2.19(brs,1H), 2.19-2.33(brs,1H), 2.41(s,3H), 3.43-3.58(brs,1H), 3.58-3.76(m,2H), 3.76-3.99(m,2H), 6.18(d,J=7.3Hz,1H), 7.05(s,1H), 7.89(d,J=14.2Hz,1H), 8.3-8.5(brs,3H), 8.76(s,1H)

### Example 44

### Synthesis of ethyl 6,7-difluoro-1-(3-methylisothiazol-4-yl)-1,4-dihydro-4-oxoquinoline-3-carboxylate (Compound No. 94)

Ethyl 2,4,5-trifluorobenzoyl acetate (720 mg), 650 mg of ethyl orthoformate and 1.35 g of acetic anhydride were stirred at 130°C for 3 hours. The reaction mixture was distilled off under reduced pressure. The residue was added with 20 mℓ of chloroform and a solution of 334 mg of 4-amino-3-methylisothiazole in 20 mℓ of methanol, followed by stirring at room temperature for one hour. The solvent was distilled off. To the residue, 30 mℓ of hexane were added, followed by collection through filtration, whereby 910 mg of a pale yellow solid was obtained. The solid so obtained was dissolved in 50 mℓ of THF. To the resulting solution, 122 mg of sodium hydride (60%, oil) were added, followed by stirring at room temperature for one hour. After an aqueous solution of citric acid was added to the reaction mixture to neutralize the same, the solvent was distilled off. The residue was extracted with 50 mℓ of chloroform. The extract was dried (MgSO₄), followed by removal of the solvent by distillation. To the residue, hexane was added. The solid so obtained was collected by filtration, whereby 770 mg of the title compound was obtained as a pale yellow solid.
- Melting point:: 264.5-268°C
- ¹H-NMR(CDCℓ₃)δ;: 1.39(t,J=7Hz,3H), 2.29(s,3H), 4.37(q,J=7Hz,2H), 6.6(dd,J=10Hz,J=6Hz,1H), 8.27(dd,J=10.3Hz,J=8.3Hz,1H), 8.35(s,1H), 8.98(s,1H)

### Example 45

### Synthesis of 6,7-difluoro-1-(3-methylisothiazol-4-yl)-1,4-dihydro-4-oxoquinoline-3-carboxylic acid (Compound No. 95)

To 25 mℓ of acetic acid and 10 mℓ of 6N hydrochloric acid, 750 mg of Compound No. 94 were added, followed by hydrolysis at 110°C for one hour. After being cooled down, the hydrolyzate was added with water. The solid so obtained was collected by filtration and then washed with water, ethanol and ethyl ether, whereby 644 mg of the title compound was obtained as a slightly yellow solid.
- Melting point:: 297-300°C
- ¹H-NMR(DMSO-d₆)δ;: 2.18 (d,J=1.4Hz,3H), 7.3-7.4(m,1H), 8.32-8.39(m,1H), 8.90(d,J=1Hz,1H), 9.51(d,J=1.4Hz,1H)

### Example 46

### Synthesis of 7-(3-(S)-aminopyrrolidin-1-yl)-6-fluoro-1-(3-methylisothiazol-4-yl)-1,4-dihydro-4-oxoquinoline-3-carboxylic acid hydrochloride (Compound No. 96)

To 5 mℓ of acetonitrile, 50 mg of Compound No. 95, 15 mg of 3-(S)-aminopyrrolidine and 40 mg of triethylamine were added, followed by stirring at 80°C for one hour. The solid so obtained was collected by filtration and then dissolved in 3 mℓ of 6N hydrochloric acid. The solvent was distilled off. The residue was suspended in ethyl ether, followed by collection through filtration, whereby 57 mg of the title compound was obtained as a pale yellow solid.
- Melting point:: 278°C or higher (decomposed)
- ¹H-NMR(DMSO-d₆)δ;: 2.0-2.35(m,2H), 2.19(s,3H), 3.2-3.95(m,5H), 5.75(d,J=7.3Hz,1H), 7.94(d,J=14.1Hz,1H), 8.28(brs,3H), 8.7(s,1H), 9.55(s,1H)

### Example 47

### Synthesis of ethyl 2-(2,4,5-trifluorobenzoyl)-3-(4-methylisothiazol-3-ylamino)acrylate (Compound No. 97)

Ethyl 2,4,5-trifluorobenzoylacetate (432 mg), 400 mg of ethyl orthoformate and 805 mg of acetic anhydride were combined, followed by heating at 130°C for 3 hours. Excess ethyl orthoformate and acetic anhydride were distilled off. The residue was dissolved in 10 mℓ of chloroform. To the resulting solution, a solution of 200 mg of 3-amino-4-methylisothiazole in 10 mℓ of methanol was added, followed by stirring overnight at room temperature. The solvent was then distilled off. The residue was purified using a silica gel column (hexane:ethyl acetate = 10:1), whereby 248 mg of the title compound was obtained as a pale yellow solid.
- ¹H-NMR(CDCℓ₃)δ;: 1.03(t,J=7Hz) and 1.20(t,J=7Hz) for 3H, 2.29(s) and 2.33(s) for 3H, 4.08-4.22(m,2H), 6.84-6.98(m,1H), 7.42-7.55(m) and 7.28-7.37(m) for 1H, 8.27(s) and 8.31(s) for 1H, 8.88(d,J=12.7Hz) and 9.09(d,J=12.7Hz for 1H)

### Example 48

### Synthesis of ethyl 6,7-difluoro-1-(4-methylisothiazol-3-yl)-1,4-dihydro-4-oxoquinoline-3-carboxylate (Compound No. 98)

In 2 mℓ of N,N-dimethylformamide, 220 mg of Compound No. 97 were dissolved. To the resulting solution, 91 mg of potassium carbonate were added, followed by stirring at 90°C for 10 minutes. The reaction mixture was poured into an aqueous solution of citric acid. The solid so obtained was collected by filtration, followed by washing with water, ethanol and diethyl ether, whereby 157 mg of the title compound was obtained as a colorless solid.
- Melting point:: 191-200°C
- ¹H-NMR(CDCℓ₃)δ;: 1.42(t,J=7Hz,3H), 2.14(d,J=1.0Hz,3H), 4.42(q,J=7Hz,2H), 6.67(dd,J=6.4Hz,J=10.7Hz,1H), 8.33(dd,J=8.3Hz,J=10.3Hz,1H), 8.46(s,1H), 8.68(d,J=1.0Hz,1H)

### Example 49

### Synthesis of 6,7-difluoro-1-(4-methylisothiazol-3-yl)-1,4-dihydro-4-oxoquinoline-3-carboxylic acid (Compound No. 99)

To 140 mg of Compound No. 98, 3 mℓ of acetic acid and 1 mℓ of 6N hydrochloric acid were added, followed by stirring at 110°C for one hour. The reaction mixture was allowed to cool down and the solid so precipitated was collected by filtration. The solid was then washed with water, ethanol and diethyl ether, whereby 112 mg of the title compound were obtained as colorless needle crystals.
- Melting point:: 224-227°C
- ¹H-NMR(DMSO-d₆)δ;: 2.05(s,3H), 7.24(dd,J=6.4Hz,11.2Hz,1H), 8.35(dd,J=8.8Hz,10.3Hz,1H), 8.96(s,1H), 9.10(s,1H)

### Example 50

In a similar manner to Example 4, Compounds Nos. 100 and 101 shown in Table 8 were synthesized using Compound No. 99.

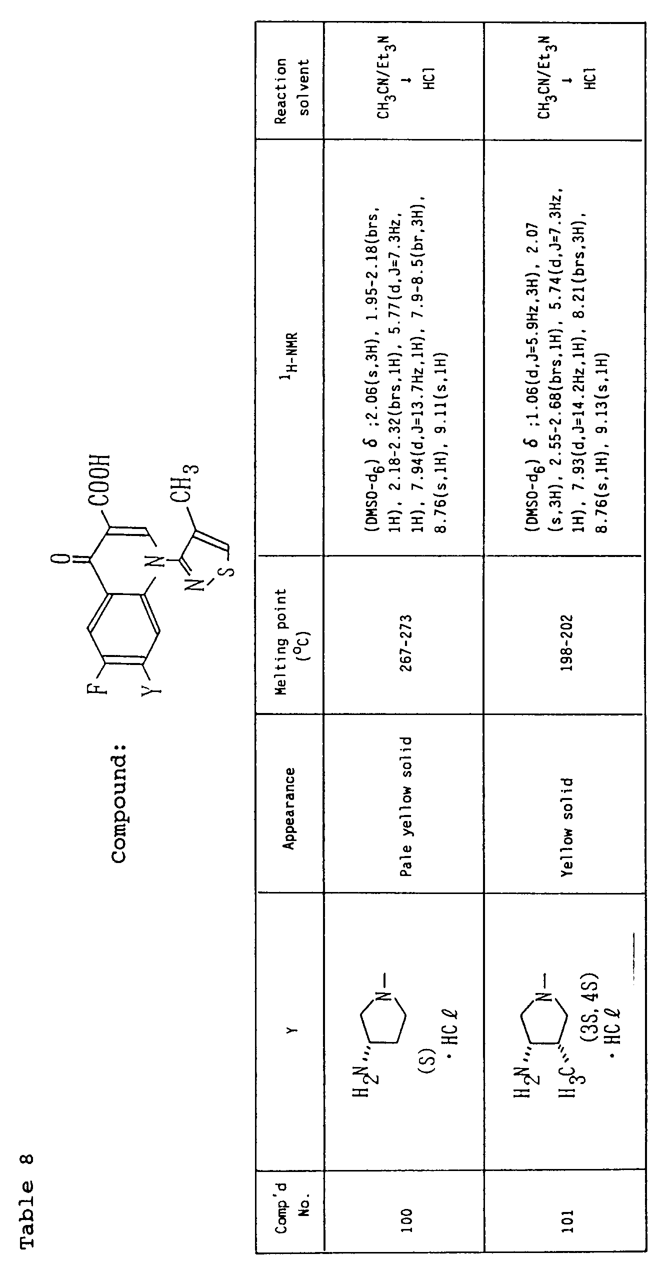

### Example 51

### Synthesis of ethyl 2-(2,4,5-trifluorobenzoyl)-3-(4-chloroisoxazol-3-ylamino)acrylate (Compound No. 102)

Ethyl 2,4,5-trifluorobenzoylacetate (1.00 g), 0.91 g of ethyl orthoformate and 1.87 g of acetic anhydride were combined, followed by stirring at 130°C for 3 hours. Excess ethyl orthoformate and acetic anhydride were distilled off. The residue was dissolved in 20 mℓ of chloroform. To the resulting solution, a solution of 482 mg of 3-amino-4-chloroisoxazole in 20 mℓ of methanol was added, followed by stirring overnight at room temperature. The solvent was then distilled off. The residue was purified using a silica gel column (hexane:ethyl acetate = 5:1), whereby 1.25 g of the title compound was obtained as a yellow oil.
- Melting point:: 87-89°C
- ¹H-NMR(CDCℓ₃)δ;: 1.06(t,J=7Hz) and 1.20(t,J=7.0Hz) for 3H, 4.16-4.23(m,2H), 6.83-7.00(m,1H), 7.31-7.42(m) and 7.48-7.59(m) for 1H, 8.38(s) and 8.42(s) for 1H, 8.44(d,J=12.7Hz) and 8.71(d,J=12.2Hz) for 1H

### Example 52

### Synthesis of ethyl 6,7-difluoro-1-(4-chloroisoxazol-3-yl)-1,4-dihydro-4-oxoquinoline-3-carboxylate (Compound No. 103)

In 5 mℓ of N,N-dimethylformamide, 1.00 g of Compound No. 102 was dissolved. To the resulting solution, 410 mg of potassium carbonate were added, followed by stirring at 90°C for 10 minutes. The reaction mixture was poured into 150 mℓ of 5% aqueous solution of citric acid. The solid so obtained was collected by filtration and then washed with water, ethanol and diethyl ether, whereby 728 mg of the title compound was obtained as a pale yellow solid.
- Melting point:: 227-237°C
- ¹H-NMR(CDCℓ₃)δ;: 1.41(t,J=7Hz,3H), 4.41(q,J=7Hz,2H), 6.98(dd,J=6.4Hz,10.8Hz,1H), 8.31(dd,J=8.8Hz,10.3Hz,1H), 8.45(s,1H), 8.83(s,1H)

### Example 53

### Synthesis of 6,7-difluoro-1-(4-chloroisoxazol-3-yl)-1,4-dihydro-4-oxoquinoline-3-carboxylic acid (Compound No. 104)

To 728 mg of Compound No. 103, 20 mℓ acetic acid and 10 mℓ of 6N hydrochloric acid were added, followed by stirring at 110°C for one hour. After being cooled down, the reaction mixture was added with water to precipitate crystals. The crystals so obtained were collected by filtration and then, washed with water, ethanol and diethyl ether, whereby 624 mg of the title compound were obtained as slightly yellow needle crystals.
- Melting point:: 215-218°C
- ¹H-NMR(DMSO-d₆)δ;: 7.82(dd,J=6.3Hz,11.2Hz,1H), 8.35(dd,J=8.3Hz,10.3Hz,1H), 9.14(s,1H), 9.76(s,1H)

### Example 54

In a similar manner to Example 4, Compounds Nos.105-107 shown in Table 9 were synthesized using Compound No. 104.

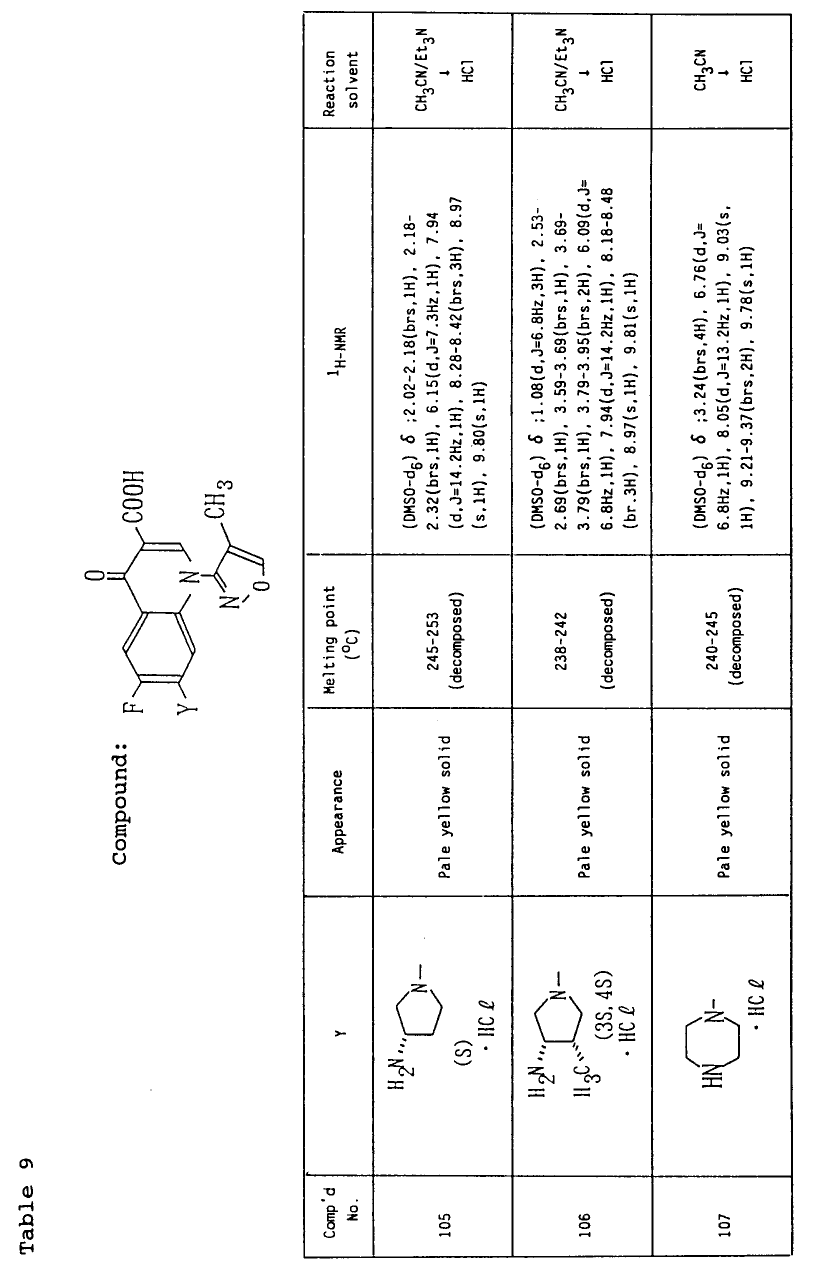

### Example 55

### Synthesis of ethyl 5-benzyloxy-6,7-difluoro-1-(isoxazol-3-yl)-1,4-dihydro-4-oxoquinoline-3-carboxylate (Compound No. 108)

Ethyl 2-benzyloxy-3,4,6-trifluorobenzoylacetate (7.04 g), 5.7 mℓ of acetic anhydride and 5 mℓ of ethyl orthoformate were stirred at 130°C for 2 hours. The reaction mixture was distilled off under reduced pressure. To the residue, 50 mℓ of chloroform and 1.68 g of 3-aminoisoxazole were added, followed by stirring at room temperature for 2 hours. The solvent was distilled off. To the residue, 30 mℓ of DMF and 2.76 g of anhydrous potassium carbonate were added, followed by stirring at 100°C for 15 minutes. After the solvent was distilled off, to the residue 100 mℓ of 5% aqueous citric acid solution were added. The solid so obtained was collected by filtration and then washed successively with ethanol and isopropyl ether, whereby 5 g of the title compound was obtained as a pale yellow solid.
- Melting point:: 171-173.5°C
- ¹H-NMR(CDCℓ₃)δ;: 1.37(t,J=7Hz,3H), 4.36(q,J=7Hz,2H), 5.26(s,2H), 6.8(d,J=2Hz,1H), 6.8-6.9(m,1H), 7.3-7.45(m,3H), 7.55-7.65(m,2H), 8.35(s,1H), 8.64(d,J=2Hz,1H)

### Example 56

### Synthesis of 6,7-difluoro-5-hydroxy-1-(isoxazol-3-yl)-1,4-dihydro-4-oxoquinoline-3-carboxylic acid (Compound No. 109)

To a mixed solvent of 20 mℓ of 6N hydrochloric acid and 15 mℓ of acetic acid, 3 g of Compound No. 108 were added, followed by stirring at 100°C for 14 hours. The solvent was distilled off under reduced pressure. To the residue, 100 mℓ of water were added. The solid so obtained was collected by filtration and then washed successively with ethanol and ethyl ether, whereby 2 g of the title compound was obtained as a slightly yellow solid.
- Melting point:: 247.5-249.5°C
- ¹H-NMR(DMSO-d₆)δ;: 6.96(dd,J=6Hz,12Hz,1H), 7.24(d,J=2Hz,1H), 8.81(s,1H), 9.35(d,J=2Hz,1H)

### Example 57

In a similar manner to Example 4, Compounds Nos. 110-114 shown in Table 10 were synthesized using Compound No. 109 and their corresponding amines.

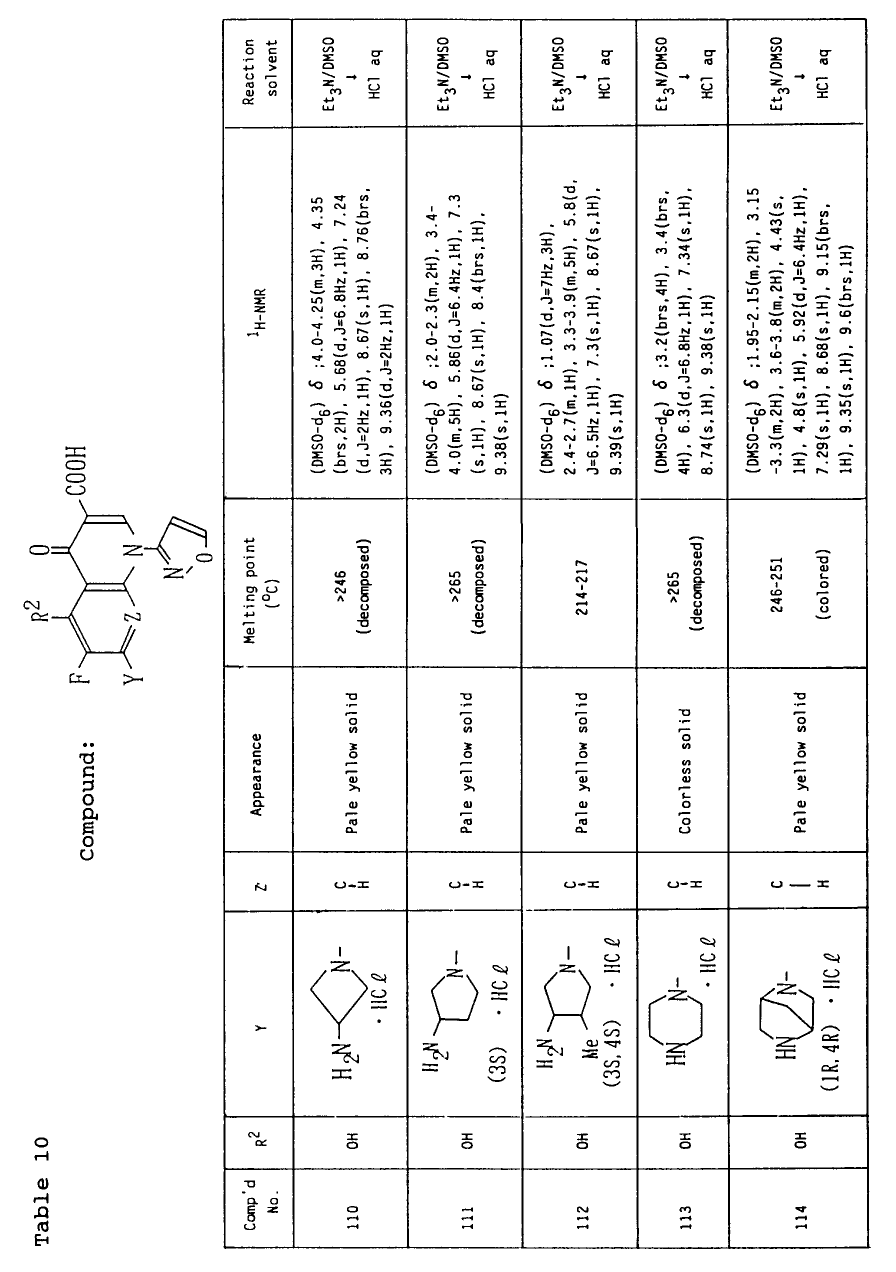

### Example 58

In a similar manner to Example 4, Compounds Nos.115-125 shown in Tables 11-1 to 11-3 were synthesized.

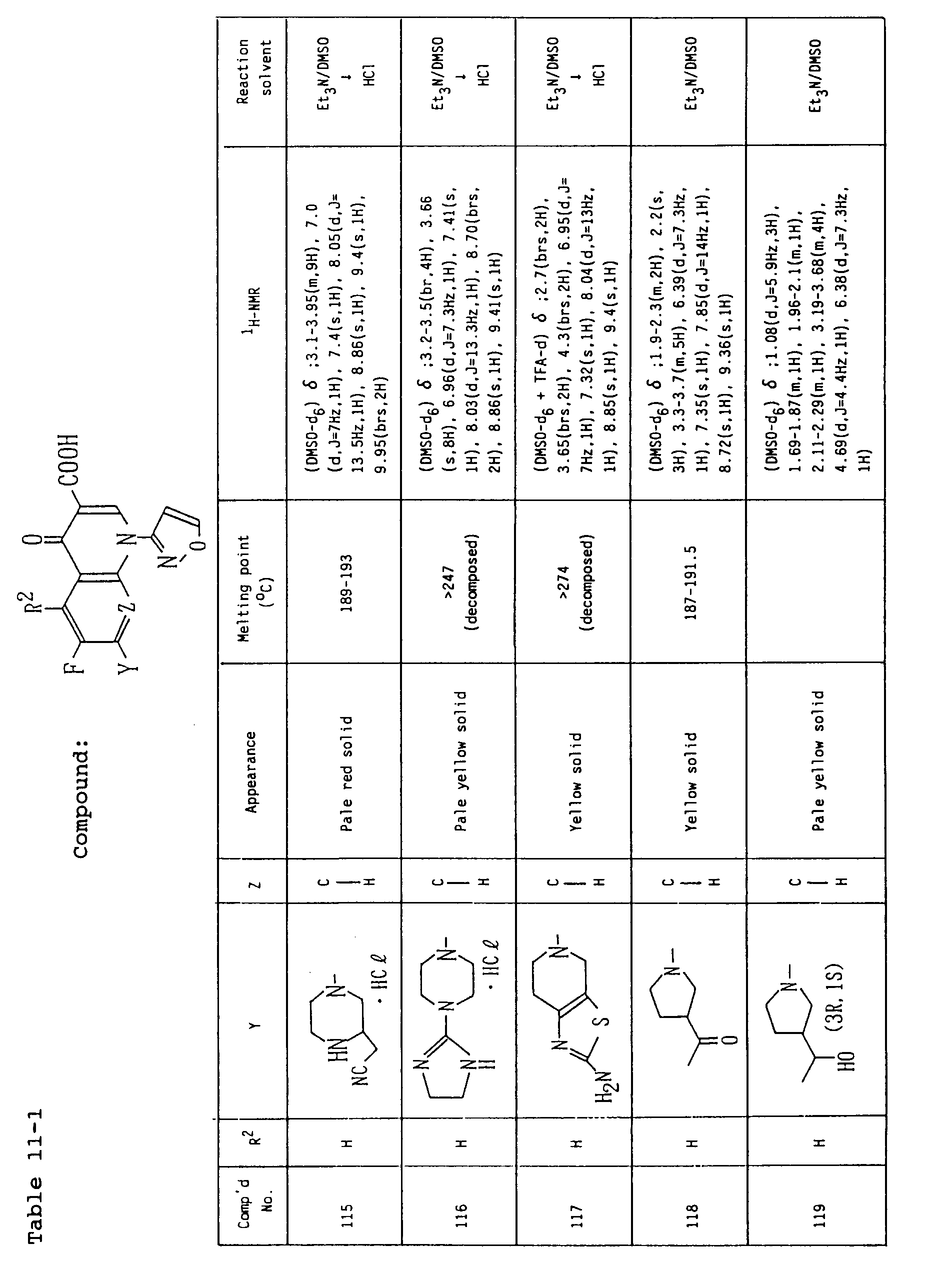

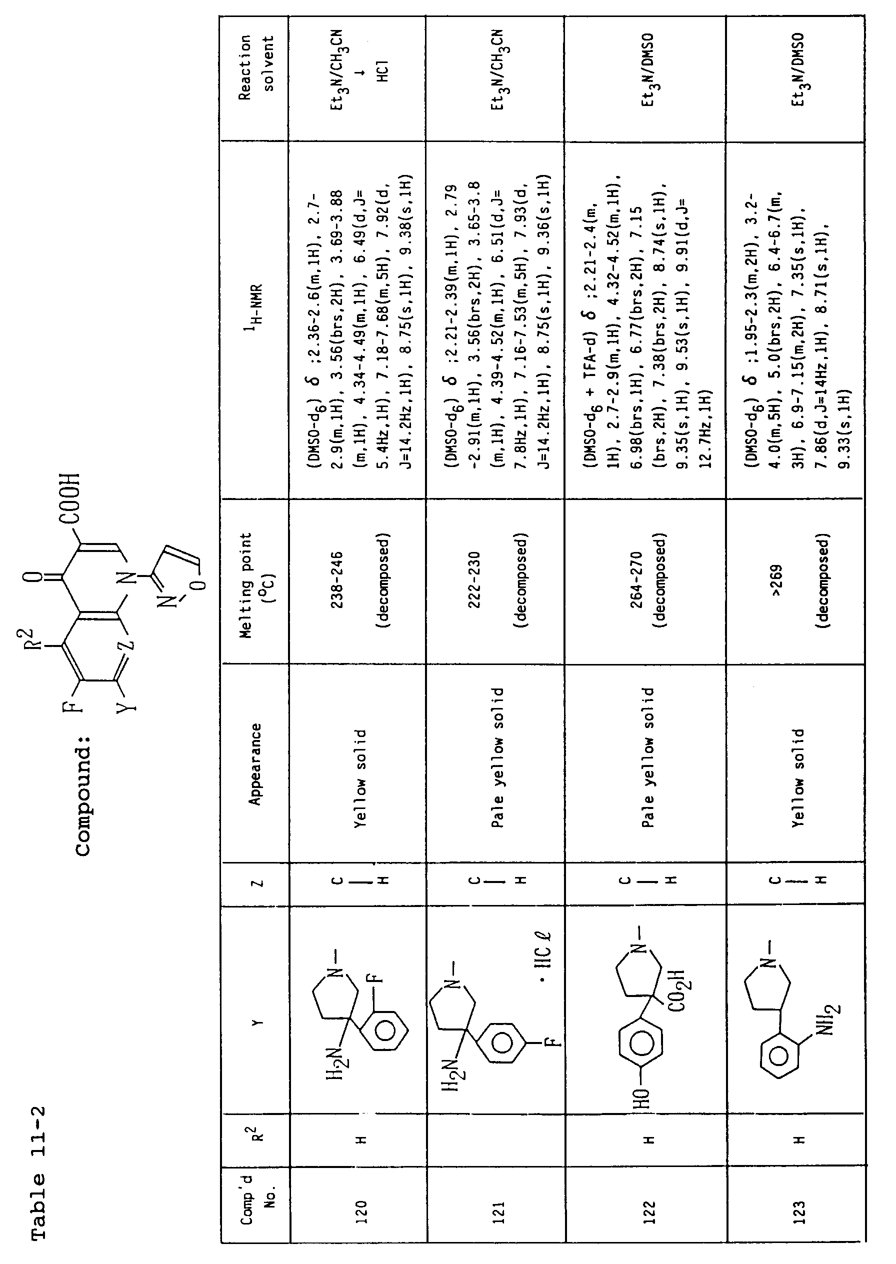

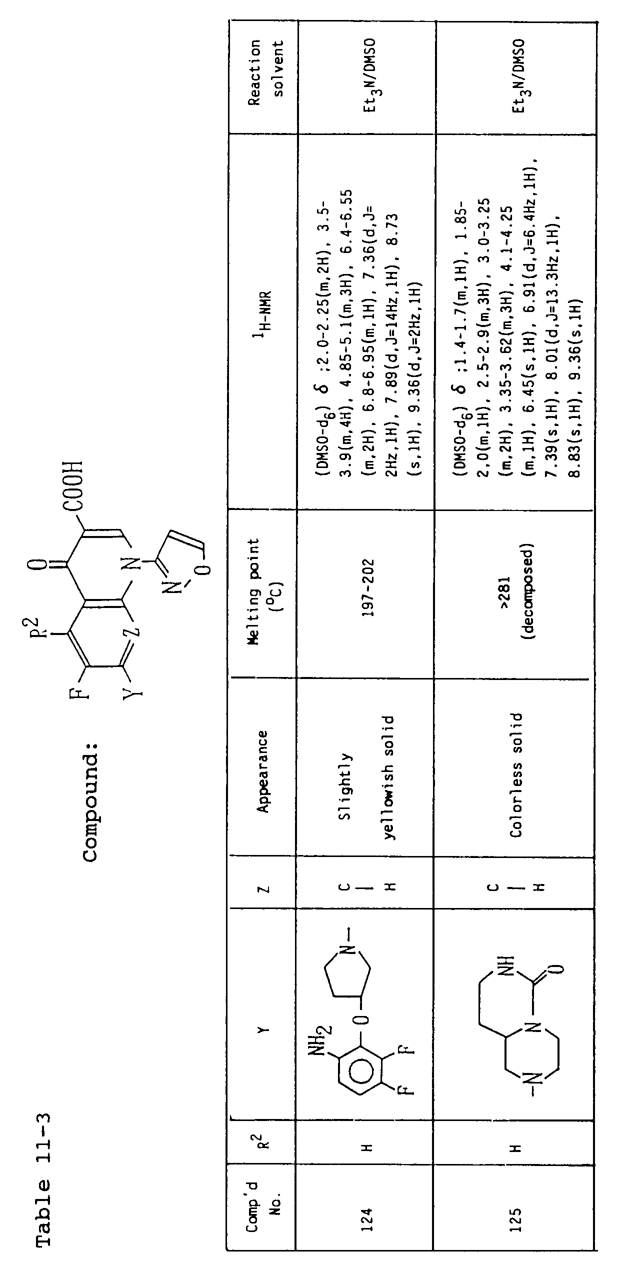

### Example 59

### Synthesis of ethyl 7-{(3S,4S)-3-amino-4-methylpyrrolidin-1-yl}-6-fluoro-1-(isoxazol-3-yl)-1,4-dihydro-4-oxoquinoline-3-carboxylate (Compound No. 126)

In 20 mℓ of N,N-dimethylsulfoxide, 300 mg of Compound No. 2 were dissolved. To the resulting solution, 184 mg of (3S,4S)-3-amino-4-methylpyrrolidine dihydrochloride and 330 mg of triethylamine were added, followed by stirring overnight at 80°C. Ethyl acetate (100 mℓ) and 100 mℓ of water were added to the reaction mixture to cause separation. The organic layer so obtained was washed with 100 mℓ of water and dried over anhydrous sodium sulfate. The solvent was distilled off, whereby 245 mg of the title compound was obtained as a yellow solid.
- Melting point:: 194-203°C
- ¹ H-NMR(CDCℓ₃)δ;: 1.09(t,J=6.8Hz,3H), 1.38(t,J=7Hz,3H), 2.25-2.39(m,1H), 3.21-3.34(m,2H), 3.45-3.58(m,2H), 3.64-3.74(m,1H), 4.36(q,J=7Hz,2H), 6.26(d,J=7.3Hz,1H), 6.79(d,J=2.0Hz,1H), 7.93(d,J=14.7Hz,1H), 8.41(s,1H), 8.69(d,J=2.0Hz,1H)

### Example 60

### Synthesis of ethyl 7-{(3S,4S)-3-(N-t-butoxycarbonylalanylamino)-4-methylpyrrolidin-1-yl}-6-fluoro-1-(isoxazol-3-yl)-1,4-dihydro-4-oxoquinoline-3-carboxylate (Compound No. 127)

In 2 mℓ of methylene chloride, 104 mg of N-t-butoxycarbonylalanine and 57 mg of triethylamine were dissolved, followed by stirring under ice cooling. To the reaction mixture, 0.08 mℓ of isobutyl chloroformate was added slowly. To the resulting mixture, a solution of 220 mg of Compound No. 126 in 4 mℓ of methylene chloride was added. The resulting mixture was allowed to rise back to room temperature, at which the mixture was stirred for 30 minutes. The reaction mixture was washed with 5% aqueous citric acid solution and 5% aqueous sodium bicarbonate solution, each 10 mℓ and was dried over anhydrous magnesium sulfate. The solvent was then distilled off. By the addition of isopropyl ether, the residue was solidified. The solid so obtained was collected by filtration, whereby 237 mg of the title compound was obtained as a pale yellow solid.
- Melting point:: 137-141°C
- ¹H-NMR(CDCℓ₃)δ;: 1.09(d,J=6.8Hz,3H), 1.13(d,J=6.4Hz,3H), 1.3(s,9H), 1.40(t,J=7Hz,3H), 2.39-2.55(brs,1H), 2.89-3.05(m,1H), 3.13-3.30(brs,1H), 3.59-3.78(m,1H), 3.88-4.01(m,1H), 4.18-4.42(m,3H), 4.62-4.75(brs,1H), 5.54-5.65(brs,1H), 6.02(d,J=6.8Hz,1H), 6.79(d,J=1.5Hz,1H), 7.55(d,J=14.2Hz,1H), 8.02-8.16(brs,1H), 8.32(s,1H), 8.69(d,J=1.5Hz,1H)

### Example 61

### Synthesis of 7-{(3S,4S)-3-(N-alanylamino)-4-methylpyrrolidin-1-yl}-6-fluoro-1-(isoxasol-3-yl)-1,4-dihydro-4-oxoquinoline-3-carboxylic acid hydrochloride (Compound No. 128)

In 15 mℓ of tetrahydrofuran, 200 mg of Compound No. 127 were dissolved. The resulting solution was added with 2 mℓ of 6N hydrochloric acid, followed by stirring at room temperature for one week. The solvent was then distilled off. The residue was suspended in diethyl ether, followed by collection through filtration, whereby 138 mg of the title compound was obtained as a pale brown solid.
- Melting point:: 197-215°C
- ¹H-NMR(DMSO-d₆)δ;: 0.97(d,J=6.8Hz,3H), 1.33(d,J=6.8Hz,3H), 4.42(brs,1H), 6.39(d,J=7.3Hz,1H), 7.34(s,1H), 7.89(d,J=14.2Hz,1H), 8.19(brs,3H), 8.56(d,J=8.3Hz,1H), 8.73(s,1H), 9.37(d,J=1.5Hz,1H)

### Example 62

### Synthesis of 7-{(3S,4S)-3-amino-4-methylpyrrolidin-1-yl}-6-fluoro-1-(isoxazol-3-yl)-1,4-dihydro-4-oxoquinoline-3-carboxylic acid (Compound No. 129)

In 80 mℓ of acetonitrile, 3.00 g of Compound No. 3 were suspended. To the resulting suspension, 1.96 g of (3S,4S)-3-amino-4-methylpyrrolidine dihydrochloride and 4.60 g of triethylamine were added, followed by stirring at 80°C for one hour. The precipitate so obtained was collected by filtration and then washed with ethanol, whereby 3.50 g of the title compound was obtained as a pale yellow solid.
- Melting point:: 222-228°C
- ¹H-NMR(DMSO-d₆)δ;: 1.00(d,J=6.7Hz,3H), 2.17-2.31(brs,1H), 6.30(d,J=7.3Hz,1H), 7.34(d,J=1.5Hz,1H), 7.80(d,J=14.2Hz,1H), 8.67(s,1H), 9.37(d,J=1.5Hz,1H)

### Example 63

### Synthesis of 7-{(3S,45)-3-amino-4-methylpyrrolidin-1-yl}-6-fluoro-1-(isoxazol-3-yl)-1,4-dihydro-4-oxoquinoline-3-carboxylic acid, 1/2 sulfuric acid salt (Compound No. 130)

In 220 mℓ of water, 7.00 g of Compound No. 129 were suspended. To the resulting suspension, 1.85 g of concentrated sulfuric acid were added, followed by heating under reflux. To the reaction mixture, water was added in portions until the compound was almost dissolved (280 mℓ in total). The resulting mixture was subjected to hot filtration. The filtrate was then allowed to cool down. The crystals so precipitated were collected by filtration and then washed with a small amount of cold water, whereby 5.61 g of the title compound were obtained as pale yellow fine needle crystals.
- Melting point:: 223-228°C
- ¹H-NMR(DMSO-d₆)δ;: 1.04(d,J=6.8Hz,3H), 2.36-2.51(brs,1H), 6.36(d,J=7.3Hz,1H), 7.34(d,J=1.5Hz,1H), 7.88(d,J=14.2Hz,1H), 8.73(s,1H), 9.38(d,J=2.0Hz,1H)

### Example 64

### Synthesis of 7-{(3S,4S)-3-amino-4-methylpyrrolidin-1-yl}-6-fluoro-1-(isoxazol-3-yl)-1,4-dihydro-4-oxoquinoline-3-carboxylic acid, acetic acid salt (Compound No. 131)

In 100 mℓ of water, 8.50 g of Compound No. 129 were suspended. The resulting suspension was heated under reflux and was added with acetic acid in small portions until it was dissolved completely. The solvent was distilled off. The residue was crystallized from 200 mℓ of ethanol and 38 mℓ of 50% acetic acid, whereby 6.44 g of the title compound were obtained as pale yellow fine crystals.
- Melting point:: 225-233°C
- ¹H-NMR(DMSO-d₆)δ;: 0.99(d,J=6.8Hz,3H), 1.90(s,3H), 2.17-2.31(br,1H), 6.32(d,J=7.8Hz,1H), 7.35(d,J=1.5Hz,1H), 7.84(d,J=14.2Hz,1H), 8.70(s,1H), 9.36(d,J=1.5Hz,1H)

### Example 65

### Synthesis of 7-{(3S,4S)-3-amino-4-methylpyrrolidin-1-yl}-6-fluoro-1-(isoxazol-3-yl)-1,4-dihydro-4-oxoquinoline-3-carboxylic acid, p-toluenesulfonic acid salt (Compound No. 132)

In 80 mℓ of water, 7.50 g of Compound No. 129 were suspended. To the resulting suspension, 3.83 g of p-toluenesulfonic acid were added, followed by heating under reflux. After the reaction mixture was dissolved completely, the solvent was distilled off. The residue was crystallized from 200 mℓ of ethanol and 38 mℓ of water, whereby 9.48 g of the title compound were obtained as pale yellow crystals.
- Melting point:: 215-220°C
- ¹H-NMR(DMSO-d₆)δ;: 1.04(d,J=6.8Hz,3H), 2.28(s,3H), 2.54-2.69(br,1H), 3.39-3.48(m,2H), 3.58-3.70(m,2H), 6.39(d,J=7.3Hz,1H), 7.10(d,J=7.8Hz,2H), 7.32(d,J=1.5Hz,1H), 7.46(d,J=8.3Hz,1H), 7.93(d,J=14.2Hz,1H), 8.77(s,1H), 9.39(d,J=2.0Hz,1H)

### Example 66

### Synthesis of 7-{(3S,4S)-3-amino-4-methylpyrrolidin-1-yl}-6-fluoro-1-(isoxazol-3-yl)-1,4-dihydro-4-oxoquinoline-3-carboxylic acid, methanesulfonic acid salt (Compound No. 133)

In 100 mℓ of water, 7.50 g of Compound No. 129 were suspended. To the resulting suspension, 1.94 g of methanesulfonic acid were added, followed by heating under reflux. After the reaction mixture was dissolved completely, the solvent was distilled off. The residue was crystallized from 200 mℓ of ethanol and 12 mℓ of water, whereby 7.63 g of the title compound were obtained as pale yellow crystals.
- Melting point:: 273-275°C
- ¹H-NMR(DMSO-d₆)δ;: 1.08(d,J=6.8Hz,3H), 2.32(s,3H), 2.52-2.69(br,1H), 3.58-3.70(brs,2H), 3.79-3.92(brs,2H), 6.40(d,J=7.3Hz,1H), 7.33(d,J=2.0Hz,1H), 7.93(d,J=14.2Hz,1H), 7.88-8.10(br,3H), 8.77(s,1H), 9.39(d,J=1.0Hz,1H)

### Example 67

### Synthesis of 7-{(3S,4S)-3-amino-4-methylpyrrolidin-1-yl}-6-fluoro-1-(isoxazol-3-yl)-1,4-dihydro-4-oxoquinoline-3-carboxylic acid, phosphoric acid salt (Compound No. 134)

In 140 mℓ of water, 7.00 g of Compound No. 129 were suspended. To the resulting suspension, 1.86 g of phosphoric acid were added, followed by heating under reflux. After the reaction mixture was dissolved, the solvent was distilled off. The residue was crystallized from 180 mℓ of ethanol and 210 mℓ of water, whereby 6.92 g of the title compound were obtained as pale yellow needle crystals.
- Melting point:: 228-232°C
- ¹H-NMR(DMSO-d₆)δ;: 1.07(d,J=6.8Hz,3H), 2.34-2.47(brs,1H), 6.33(d,J=7.3Hz,1H), 7.35(s,1H), 7.87(d,J=14.2Hz,1H), 8.72(s,1H), 9.36(s,1H)

### Example 68

### Synthesis of 7-{(3S,4S)-3-amino-4-methylpyrrolidin-1-yl}-6-fluoro-1-(isoxazol-3-yl)-1,4-dihydro-4-oxoquinoline-3-carboxylic acid, maleic acid salt (Compound No. 135)

In 100 mℓ of water, 7.00 g of Compound No. 129 were suspended. To the resulting suspension, 2.22 g of maleic acid were added, followed by heating under reflux. Water was added to the reaction mixture. When the total volume became 200 mℓ, the resulting mixture was filtered while being hot, followed by the addition of a small amount of ethanol. The resulting mixture was allowed to cool down to precipitate crystals. Crystals so precipitated were collected by filtration, whereby 7.54 g of the title compound were obtained as pale yellow crystals.
- Melting point:: 190-194°C
- ¹H-NMR(DMSO-d₆)δ;: 1.08(d,J=6.8Hz,3H), 2.52-2.69(br,1H), 3.56-3.69(brs,2H), 3.79-3.99(brs,2H), 6.01(s,2H), 6.34(d,J=7.3Hz,1H), 7.32(d,J=1.5Hz,1H), 7.92(d,J=13.7Hz,1H), 8.76(s,1H), 9.39(d,J=1.5Hz,1H)

### Example 69

### Synthesis of 7-{(3S,4S)-3-amino-4-methylpyrrolidin-1-yl]-6-fluoro-1-(isoxazol-3-yl)-1,4-dihydro-4-oxoquinoline-3-carboxylic acid, fumaric acid salt (Compound No. 136)

In 100 mℓ of water, 7.00 g of Compound No. 129 were suspended. To the resulting suspension, 2.22 g of fumaric acid were added, followed by heating under reflux. Water was added to the reaction mixture. When the total volume became 250 mℓ, filtration was conducted while being hot. The solvent was then distilled off. The residue was crystallized from 50 mℓ of water and 50 mℓ of ethanol, whereby 6.79 g of the title compound were obtained as pale yellow crystals.
- Melting point:: 193-199°C
- ¹H-NMR(DMSO-d₆)δ;: 1.05(d,J=6.3Hz,3H), 2.38-2.52(brs,1H), 3.32(t,J=8.8Hz,1H), 3.57(brs,2H), 3.67(brs,1H), 3.74(brs,1H), 6.34(d,J=7.8Hz,1H), 6.43(s,2H), 7.33(s,1H), 7.86(d,J=14.2Hz,1H), 8.72(s,1H), 9.37(d,J=1.0Hz,1H)

### Example 70

### Synthesis of 7-{(3S,4S)-3-amino-4-methylpyrrolidin-1-yl}-6-fluoro-1-(isoxazol-3-yl)-1,4-dihydro-4-oxoquinoline-3-carboxylic acid, sodium salt (Compound No. 137)

To 500 mg of Compound No. 129, 2.5 mℓ of a 0.5 N solution of sodium hydroxide were added, followed by filtration. The solvent was distilled off at low temperature. The residue was crystallized from water, whereby 242 mg of the title compound were obtained as colorless needle crystals.
- Melting point:: 228-231°C
- ¹H-NMR(DMSO-d₆)δ;: 0.99(d,J=6.8Hz,3H), 2.16-2.31(brs,1H), 6.29(d,J=7.8Hz,1H), 7.33(d,J=1.5Hz,1H), 7.79(d,J=14.2Hz,1H), 8.65(s,1H), 9.34(d,J=2.0Hz,1H)

### Example 71

### Synthesis of 5-amino-6-fluoro-7-{(3S,4S)-3-t-butoxycarbonylamino-4-methylpyrrolidin-1-yl}-1-(isoxazol-3-yl)-1,4-dihydro-4-oxoquinoline-3-carboxylic acid (Compound No. 138)

In 2 mℓ of DMSO, 160 mg of Compound No. 56, 192 mg of (3S,4S)-3-t-butoxycarbonylamino-4-methylpyrrolidine formate and 200 mg of triethylamine were dissolved, followed by stirring at 80°C for 2 hours. To the reaction mixture, 150 mℓ of ethyl acetate were added, followed by washing with 30 mℓ of 5% aqueous citric acid solution and then twice with 30 mℓ of water. After drying over MgSO₄, the solvent was distilled off. The residue was dispersed in isopropyl ether, followed by collection through filtration, whereby 150 mg of the title compound was obtained as a yellow solid.
- ¹H-NMR(CDCℓ₃)δ;: 1.08(d,J=6.7Hz,3H), 1.43(s,9H), 2.35-2.55(m,1H), 3.1-3.25(m,1H), 3.4-3.75(m,3H), 4.25(brs,1H), 5.1-5.25(m,1H), 5.51(d,J=7.2Hz,1H), 6.77(s,1H), 8.37(s,1H), 8.69(d,J=2Hz,1H)

### Example 72

### Synthesis of {(1-(cyclohexyloxycarbonyloxy)ethyl} 5-amino-6-fluoro-7-{(3S,4S)-3-t-butoxycarbonylamino-4-methylpyrrolidin-1-yl}-1-(isoxazol-3-yl)-1,4-dihydro-4-oxoquinoline-3-carboxylate (Compound No. 139)

In 10 mℓ of DMF, 290 mg of Compound No. 138, 317 mg of cesium carbonate and 159 mg of 1-chloro-1-cyclohexyloxycarbonyloxyethane were dissolved, followed by stirring at room temperature for 24 hours. To the reaction mixture, 100 mℓ of ethyl acetate were added, followed by washing with a 5% aqueous solution of citric acid and water. After drying over MgSO₄, the solvent was distilled off. The residue was subjected to column chromatography (SiO₂,CHCℓ₃/AcOET 1/1), whereby 300 mg of the title compound was obtained as a yellow solid.
- ¹H-NMR(CDCℓ₃)δ;: 0.8-2.0(m,10H), 1.05(d,J=6.8Hz,3H), 1.45(s,9H), 1.6(d,J=5.5Hz,3H), 2.35-2.55(m,1H), 3.1-3.7(m,4H), 4.25(brs,1H), 4.6-4.8(m,2H), 5.43(d,J=7.2Hz,1H), 6.72(s,1H), 6.96(q,J=5.4Hz,1H), 8.24(s,1H), 8.67(s,1H)

### Example 73

### Synthesis of {1-(cyclohexyloxycarbonyloxy)ethyl} 5-amino-6-fluoro-7-{(3S,4S)-3-amino-4-methyl-pyrrolidin-1-yl}-1-(isoxazol-3-yl)-1,4-dihydro-4-oxoquinoline-3-carboxylate, hydrochloride (Compound No. 140)

In 10 mℓ of chloroform, 450 mg of Compound No. 139 were dissolved. To the resulting solution, 1 mℓ of 4N hydrochloric acid/dioxane was added, followed by stirring at room temperature for 1.5 hours. After the solvent was distilled off, the residue was solidified by adding isopropyl ether. The solid so obtained was collected by filtration, whereby 330 mg of the title compound was obtained as a yellow solid.
- Melting point:: 190.5°C or higher (decomposed)
- ¹H-NMR(DMSO-d₆)δ;: 0.9-1.9(m,16H), 2.45-2.6(m,1H), 3.2-3.8(m,5H), 4.55(brs,1H), 5.37(d,J=6Hz,1H), 6.75(q,J=5.5Hz,1H), 7.23(s,1H), 8.30(s,1H), 8.39(brs,3H), 9.34(s,1H)

### Example 74

### Synthesis of ethyl 5-amino-6-fluoro-7-{(3S,4S)-3-(2-t-butoxycarbonylamino)propionylamino-4-methyl-pyrrolidin-1-yl}-1-(isoxazol-3-yl)-1,4-dihydro-4-oxoquinoline-3-carboxylate (Compound No. 141)

In a similar manner to Examples 59 and 60, the title compound was obtained as a pale yellow solid by using Compound No. 55.
- Melting point:: 146-149°C
- ¹H-NMR(CDCℓ₃)δ;: 1.0-1.45(m,18H), 2.35-2.55(m,1H), 2.85-2.95(m,1H), 3.15-3.25(m,1H), 3.5-3.7(m,2H), 3.9(brs,1H), 4.15-4.4(m,3H), 4.65(brs,1H), 5.29(d,J=7Hz,1H), 5.75(brs,1H), 6.4-6.8(br,2H), 6.75(d,J=2Hz,1H), 7.95(brs,1H), 8.18(s,1H), 8.64(d,J=2Hz,1H)

### Example 75

### Synthesis of 5-amino-6-fluoro-7-{(3S,4S)-3-(2-amino)propionylamino-4-methylpyrrolidin-1-yl}-1-(isoxazol-3-yl)-1,4-dihydro-4-oxoquinoline-3-carboxylic acid, hydrochloride (Compound No. 142)

In a similar manner to Example 61, the title compound was obtained as a pale yellow solid by using Compound No. 141.
- Melting point:: 215-216°C
- ¹H-NMR(DMSO-d₆)δ;: 0.95(d,J=6.4Hz,3H), 1.33(d,J=6.8Hz,3H), 2.35-2.6(m,1H), 3.1-3.9(m,5H), 4.35(brs,1H), 5.55(d,J=7Hz,1H), 7.26(s,1H), 7.2-7.4(br,2H), 8.25(brs,3H), 8.52(s,1H), 8.4-8.6(br,1H), 9.34(s,1H)

### Example 76

In a similar manner to Example 4, Compounds No. 143 shown in Table 12 was synthesized using Compound No. 56.

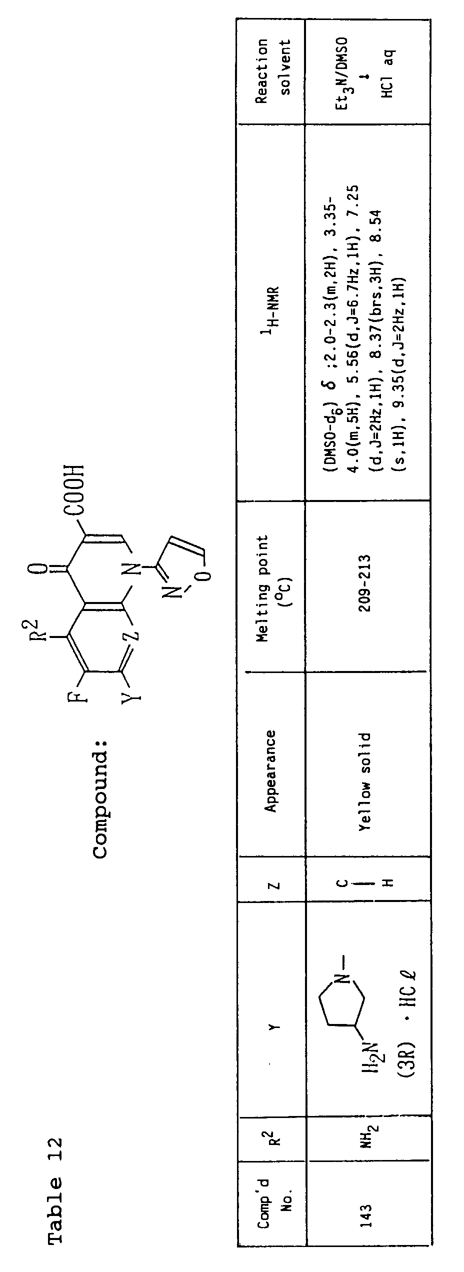

### Example 77

Antibacterial activities, absorption·excretion and photo-toxicity of the compounds obtained in the above Examples were tested as follows.

### (1) Antibacterial activities

The minimum inhibitory concentration [MIC: µg/mℓ] was measured in accordance with the standard method of Japan Society of Chemotherapy [CHEMOTHERAPY, **29**, No.1, 76-79(1981)]. The results are shown in Table 13 in which compound numbers are the same as those synthesized in the above Examples.

**Table 13**

| Comp'd No. | Minimum inhibitory concentration (µg/ml) | | |
|---|---|---|---|
| | *E*.*coli* NIH JC-2 (IFO*12734) | *S*.*aureus* 209P (IFO 12732) | *P*.*aeruginosa* (IFO 3445) |
| 4 | 0.025 | 0.1 | 0.1 |
| 5 | 0.025 | 0.1 | 0.2 |
| 49 | 0.05 | 0.2 | 0.2 |
| 57 | 0.013 | 0.05 | 0.1 |
| 58 | 0.013 | 0.05 | 0.2 |
| Ofloxacin | 0.1 | 0.39 | 1.56 |

| | | | |
|---|---|---|---|
| Note) *IFO: Institute of Fermentation, Osaka | | | |

### (2) Absorption and excretion

The absorption and excretion of the orallyadministered invention compound were tested by measuring the recovery in urine and that in bile according to the following method.

### (a) Recovery in urine

To a group of three, male, 6-week old, JCL-SD rats which had been fasted overnight, a test compound, which had been prepared to be 20 mg/10 mℓ/kg with a 0.5% methyl cellulose solution, was orally administered through an oral feeding tube. The sampling was conducted by collecting urine in 0 to 6 hours and 6 to 24 hours. The concentration of the test compound in the urine sample was examined by a thin-layer disk method which uses *Bacillus subtilis* ATCC6633 as a test bacterium, whereby the excretion rate in urine for 24 hours was determined.

### (b) Recovery in bile

After the test compound was prepared in a similar manner to the measurement of the recovery in urine, it was orally administered to the rats who had had their choledoch inserted with a polyethylene tube. The bile was collected over 24 hours. The concentration of the test compound in the bile was examined in the similar manner to the above and the excretion rate in the bile for 24 hours was determined.

The results so obtained are shown in Table 14.

**Table 14**

| Comp'd No. | Excretion rate (24 hrs, %) | |
|---|---|---|
| | in urine | in bile |
| 5 | 23.5 | 1.2 |

As described above, each of the invention compounds (1) and the salts thereof is a novel compound, exhibits excellent antibacterial activities against gram-negative and gram-positive bacteria and has high oral absorbability.

### (3) Photo-toxicity

After 40 mg/kg/10 mℓ of the test compound were administered intravenously to a female ICR mouse (5-6 week old), it was exposed to ultraviolet rays (320-400 nm, 1.8 mW/cm²/sec) for 4 hours. Twenty-four (24) and 48 hours later, said hours having been counted taking the time just after the exposure as 0 hour, its ears were observed for any abnormality. The abnormality on the ears was ranked as 0 point in the case of no abnormality, 1 point in the case of slight erythema, 2 points in the case of medium erythema, and 3 points in the case of heavy erythema or edema. The results so obtained are shown in Table 15.

**Table 15**

| Test compound | Number of toxicity-developed cases (ranking points) | | |
|---|---|---|---|
| | 0 hour | 0 hour | 0 hour |
| Compound No.4 | 0/6(0) | 0/6(0) | 0/6(0) |
| Compound No.5 | 0/6(0) | 0/6(0) | 0/6(0) |
| Enoxacin | 8/8(1.0) | 3/8(0.4) | 2/8(0.3) |
| Norfloxacin | 2/2(1.0) | 2/2(1.0) | 0/2(0) |

### Industrial Applicability

The compounds (1) according to the present invention and salts thereof are extremely useful as antibacterial agents. They can be used as not only pharmaceuticals for human bodies and animals but also medicines for fishes, agricultural chemicals and preservatives for foods. Furthermore, the compounds of the present invention are expected to have anti-viral activities, especially anti-HIV (human immuno deficiency virus) activities, and therefore is considered to have a preventive or curative effect against the AIDS.

## Claims

1. A quinolone derivative represented by the following formula (1): 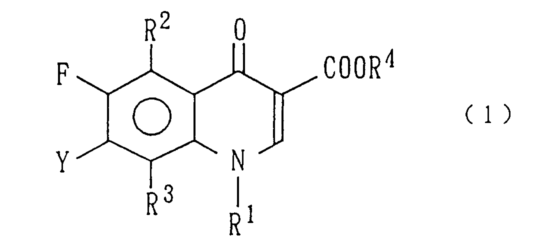 wherein R¹ represents a substituted or unsubstituted isoxazolyl group or a substituted or unsubstituted isothiazolyl group, R² represents a hydrogen atom, a halogen atom, a lower alkyl group, a hydroxyl group, an amino group or a nitro group, R³ represents a hydrogen atom or a halogen atom, R⁴ represents a hydrogen atom or a carboxyl-protecting group, and Y represents a halogen atom, a substituted or unsubstituted saturated cyclic amino group or a group H₂N-(CH₂)ₘ-A- in which A represents an oxygen atom or a sulfur atom and m stands for 0-3; or a salt thereof.

2. A quinolone derivative or a salt thereof according to claim 1, wherein R¹ represents an isoxazolyl or isothiazolyl group which may be substituted by one or more of C₁₋₇ alkyl groups, phenyl groups, halogen atoms, hydroxy groups, C₁₋₇ alkoxy groups, amino groups or nitro groups.

3. An antibacterial agent comprising as an effective ingredient the quinolone derivative or the salt thereof according to claim 1.

4. A pharmaceutical composition comprising the quinolone derivative or the salt thereof according to claim 1 and a pharmaceutically-acceptable carrier.
